# EUROPEAN PATENT SPECIFICATION

(11) **EP 3 253 377 B1**
(45) Date of publication and mention of the grant of the patent: **12.05.2021**
(21) Application number: 15760508.0
(22) Date of filing: 10.03.2015
(51) Int. Cl.: A61K 9/28, A61K 9/48, A61K 9/50, A61K 9/20, A61K 31/4015, A61K 31/397, A61K 31/40, A61K 31/403, A61K 31/4035, A61P 1/00, A61P 17/06, A61P 37/02, A61P 37/06, A61P 9/10, A61P 21/00, A61P 25/00, A61P 25/28

(54) **MONOMETHYLFUMARATE PRODRUG COMPOSITIONS**
MONOMETHYLFUMARAT-PRODRUG-ZUSAMMENSETZUNGEN
COMPOSITIONS DE PROMÉDICAMENTS À BASE DE FUMARATE DE MONOMÉTHYLE

(30) Priority: 08.02.2015 US 201562113496 P
(43) Date of publication of application: 13.12.2017
(73) Proprietor: Alkermes Pharma Ireland Limited, Dublin 4 (IE)
(72) Inventor: MANSER, David, S., Keenagh Country Longford (IE); SHAH, Hardik, Kirtikumar, Lucan Dublin (IE); PERKIN, Kristopher, K., Athlone County Roscommon (IE); BROWNING, Ivan, Athlone County Westmeath (IE)
(74) Representative: Harris, Jennifer Lucy
(86) International application number: PCT/IB2015/000731
(87) International publication number: WO 2016/124960

(56) References cited:
- WO-A1-2013/119677
- WO-A2-2006/037342
- US-A1- 2014 275 048
- US-B1- 6 359 003

## Description

### RELATED APPLICATIONS

This application claims priority to U.S. Provisional Application Serial No. 62/113,496 filed February 8, 2015.

### BACKGROUND OF THE INVENTION

Fumaric acid esters (FAEs) are approved in Germany for the treatment of psoriasis, are being evaluated in the United States for the treatment of psoriasis and multiple sclerosis, and have been proposed for use in treating a wide range of immunological, autoimmune, and inflammatory diseases and conditions.

FAEs and other fumaric acid derivatives have been proposed for use in treating a wide-variety of diseases and conditions involving immunological, autoimmune, and/or inflammatory processes including psoriasis (Joshi and Strebel, WO 1999/49858; U.S. Pat. No. 6,277,882; Mrowietz and Asadullah, Trends Mol Med 2005, 111(1), 43-48; and Yazdi and Mrowietz, Clinics Dermatology 2008, 26, 522-526); asthma and chronic obstructive pulmonary diseases (Joshi et al., WO 2005/023241 and US 2007/0027076); cardiac insufficiency including left ventricular insufficiency, myocardial infarction and angina pectoris (Joshi et al., WO 2005/023241; Joshi et al., US 2007/0027076); mitochondrial and neurodegenerative diseases such as Parkinson's disease, Alzheimer's disease, Huntington's disease, retinopathia pigmentosa and mitochondrial encephalomyopathy (Joshi and Strebel, WO 2002/055063, US 2006/0205659, U.S. Pat. No. 6,509,376, U.S. Pat. No. 6,858,750, and U.S. Pat. No. 7,157,423); transplantation (Joshi and Strebel, WO 2002/055063, US 2006/0205659, U.S. Pat. No. 6,359,003, U.S. Pat. No. 6,509,376, and U.S. Pat. No. 7,157,423; and Lehmann et al., Arch Dermatol Res 2002, 294, 399-404); autoimmune diseases (Joshi and Strebel, WO 2002/055063, U.S. Pat. No. 6,509,376, U.S. Pat. No. 7,157,423, and US 2006/0205659) including multiple sclerosis (MS) (Joshi and Strebel, WO 1998/52549 and U.S. Pat. No. 6,436,992; Went and Lieberburg, US 2008/0089896; Schimrigk et al., Eur J Neurology 2006, 13, 604-610; and Schilling et al., Clin Experimental Immunology 2006, 145, 101-107); ischemia and reperfusion injury (Joshi et al., US 2007/0027076); AGE-induced genome damage (Heidland, WO 2005/027899); inflammatory bowel diseases such as Crohn's disease and ulcerative colitis; arthritis; and others (Nilsson et al., WO 2006/037342 and Nilsson and Muller, WO 2007/042034).

FUMADERM, an enteric coated tablet containing a salt mixture of monoethyl fumarate and dimethyl fumarate (DMF) which is rapidly hydrolyzed to monomethyl fumarate, regarded as the main bioactive metabolite, was approved in Germany in 1994 for the treatment of psoriasis. FUMADERM exhibits a high degree of interpatient variability with respect to drug absorption and food strongly reduces bioavailability. Absorption is thought to occur in the small intestine with peak levels achieved 5-6 hours after oral administration. Significant side effects occur in 70-90% of patients (Brewer and Rogers, Clin Expt'l Dermatology 2007, 32, 246-49; and Hoefnagel et al., Br J Dermatology 2003, 149, 363-369). Side effects of current FAE therapy include gastrointestinal upset including nausea, vomiting, diarrhea and/or transient flushing of the skin.

Dimethyl fumarate (DMF) is the active component of the marketed drug, TECFIDERA® (Biogen), approved for the treatment of patients with relapsing forms of multiple sclerosis (MS). In a Phase IIb RRMS study, BG-12 (DMF) significantly reduced gadolinium-enhancing brain lesions. In preclinical studies, DMF administration has been shown to inhibit CNS inflammation in murine and rat EAE. It has also been found that DMF can inhibit astrogliosis and microglial activations associated with EAE. *See, e.g.,* US Published Application No. 2012/0165404.

Dimethyl fumarate is also associated with significant drawbacks. For example, dimethyl fumarate is known to cause side effects upon oral administration, such as flushing and gastrointestinal events including, nausea, diarrhea, and/or upper abdominal pain in subjects. *See, e.g.,* Gold et al., N. Eng. J. Med., 2012, 367(12), 1098-1107. Dimethyl fumarate is dosed BID with a total daily dose of about 480 mg.

Because of the disadvantages of dimethyl fumarate described above, there continues to be a need to reduce side effects and/or improve the physicochemical properties associated with DMF. There remains, therefore, a real need in the treatment of neurological diseases, such as MS, for a product which retains the pharmacological advantages of DMF but overcomes its flaws in formulation and/or adverse effects upon administration.

US 2014/0275048 discloses in its title "prodrugs of fumarates and their use in treating various diseases".

### BRIEF DESCRIPTION OF THE DRAWINGS

Figure 1 is a process flow diagram for encapsulated delayed release mini-tablets.
Figure 2 is a process flow diagram for encapsulated extruded spheres.
Figure 3 depicts a dissolution plot for encapsulated delayed release mini-tablets of 2-(2,5-dioxopyrrolidin-1-yl)ethyl methyl fumarate (Compound 1), based on a first formulation.
Figure 4 depicts dissolution plots for encapsulated delayed release mini-tablets of 2-(2,5-dioxopyrrolidin-1-yl)ethyl methyl fumarate (Compound 1), based on further formulations.
Figure 5 depicts dissolution plots for encapsulated delayed release extruded spheres of 2-(2,5-dioxopyrrolidin-1-yl)ethyl methyl fumarate (Compound 1).
Figure 6 depicts the plasma monomethyl fumarate concentration following a single oral administration of compound 1 across a range of dosage amounts.
Figure 7 depicts the dose dependent increase of Cₘₐₓ of monomethyl fumarate in plasma following administration of a pharmaceutical composition comprising 2-(2,5-dioxopyrrolidin-1-yl)ethyl methyl fumarate.
Figure 8 shows the dose dependent increase of AUCₗₐₛₜ of monomethyl fumarate in plasma following administration of a pharmaceutical composition comprising 2-(2,5-dioxopyrrolidin-1-yl)ethyl methyl fumarate.
Figure 9 depicts the monomethyl fumarate exposure in subjects dosed with 420 mg 2-(2,5-dioxopyrrolidin-1-yl)ethyl methyl fumarate or 240 mg of dimethyl fumarate.
Figure 10 depicts the maximum plasma concentration of monomethyl fumarate (Cₘₐₓ) for subjects administered a pharmaceutical composition of 420 mg 2-(2,5-dioxopyrrolidin-1-yl)ethyl methyl fumarate or 240 mg of dimethyl fumarate.
Figure 11 depicts the area under the curve of plasma monomethyl fumarate (AUCₗₐₛₜ) for subjects administered a pharmaceutical composition of 420 mg 2-(2,5-dioxopyrrolidin-1-yl)ethyl methyl fumarate or 240 mg of dimethyl fumarate.

### SUMMARY OF THE INVENTION

The present invention is as defined in the claims.

The pharmaceutical compositions of the invention comprise 2-(2,5-dioxopyrrolidin-1-yl)ethyl methyl fumarate, or a pharmaceutically acceptable salt thereof (MMF prodrug) and a controlled release polymer, wherein the controlled release polymer is in the form of a coating applied to a core (or cores) containing the prodrug. The compositions of the invention are suitable for once or twice daily administration of a monomethyl fumerate (MMF) prodrug and for use in treatment of conditions for which MMF and / or MMF prodrugs are indicated, such as for example multiple scerlosis and psoriasis. The core(s) may be in the form of one or more tablets or one or more pellets comprising the MMF prodrug.

Preferably the controlled release coating is an enteric coating which serves to protect the core from gastric fluids and only releases the prodrug once the composition has passed from the stomach into the higher pH regions of the gastrointestinal tract. Irritation of the stomach lining, which may be observed with some MMF prodrugs can thus be avoided by delaying release until composition reaches the small intestine or lower parts of the GIT. The controlled release (CR) polymer may be present in any amount which provides effective enteric protection whilst also permitting efficient release of the prodrug after the composition has passed through the stomach. For example the CR polymer is typically applied to core(s) at a level of from about 2 to about 30 % weight gain (i.e., weight gain following application of the coating solution / suspension calculated on the basis of CR polymer and any coating excipients present, such as plasticizers, anti-tack agents, etc.). Alternatively, the coating level may be defined in terms of weight per unit area and the coating (CR polymer and any coating excipients present) is typically applied to the core(s) at a level of from about 0.95 to about 14.75 mg/cm². As a further alternative, the controlled release coating may be defined in terms of its thickness and is typically applied to the core(s) to achieve a coating thickness of from about 40 to about 60 µm (microns).

Preferably substantially all, if not 100 %, of the prodrug of Formula (I), for example 2-(2,5-dioxopyrrolidin-1-yl)ethyl methyl fumarate, or a pharmaceutically acceptable salt thereof is released rapidly, for example within about 3 hours, or preferably within about 2 hours, following removal of the enteric coating (when the composition encounters a pH of about 6.8 or higher). For example, substantially all of the prodrug is released from the composition within about 2 hours in pH 6.8 as measured using USP apparatus I 40-mesh basket at a rotation speed of from 100 to 150 rpm. It is preferred that 100 % of the prodrug is released from the composition within about 2 hours in pH 6.8 as measured using USP apparatus I 40-mesh basket at a rotation speed of from 100 to 150 rpm.

In one embodiment a pharmaceutical composition according to the invention comprises a core comprising 2-(2,5-dioxopyrrolidin-1-yl)ethyl methyl fumarate, or a pharmaceutically acceptable salt thereof, a diluent and a disintegrant; and a coating comprising a controlled release polymer applied to said prodrug core.

In another embodiment, a pharmaceutical composition according to the invention comprises 2-(2,5-dioxopyrrolidin-1-yl)ethyl methyl fumarate, or a pharmaceutically acceptable salt thereof dispersed throughout a carrier matrix to form a plurality of pellets. Said pellets may be produced by melt extrusion, and subsequently coated with the controlled release polymer.

Preferably the coating used to coat the core(s) further comprises a plastizier and one or more anti-tack agents, as well as the CR polymer.

The present invention also provides solid oral dosage forms comprising a plurality of the above described controlled release tablets or pellets comprising 2-(2,5-dioxopyrrolidin-1-yl)ethyl methyl fumarate, or a pharmaceutically acceptable salt thereof, presented in a capsule. Such dosage forms have advantages over a monolithic tablet in that they facilitate the dispersal of the prodrug containing cores along the GIT, as opposed to presenting the prodrug at a single site as in the case of a monolith. It is believed that such a dispersal effect can help to minimize or reduce the occurance of gastric irritancy.

Solid oral dosage forms of the invention, as described above, have been found to have advantageous pharmacokinetic characteristics upon administration to a human subject. For example such dosage form can provide one or more of the following pharmacokinetic parameters:
i) a mean monomethyl fumarate Cₘₐₓ of from about 1.8 µg/mL to about 2.5 µg/mL in the plasma of the subject;
ii) a mean monomethyl fumarate AUCₗₐₛₜ of from about 4.0 µg·hr/mL to about 5.0 µg·hr/mL in the plasma of the subject;
iii) a median monomethyl fumarate Tₘₐₓ of from about 2.75 hours to about 3.5 hours in the plasma of the subject;
iv) a median monomethyl fumarate terminal elimination half-life (t_{1/2}) of from about 0.65 hours to about 0.8 hours in the plasma of the subject; and
v) a median monomethyl fumarate T_{lag} for absorption of from about 1 hour to about 2 hours following administration.

### DETAILED DESCRIPTION

Disclosed herein are controlled release pharmaceutical compositions comprising a compound of Formula (I), or a pharmaceutically acceptable salt thereof. The pharmaceutical compositions are useful for the treatment of a neurological disease in a subject in need thereof. In an embodiment, the neurological disease is multiple sclerosis. Also disclosed herein are controlled release pharmaceutical compositions comprising a compound of Formula (I), or a pharmaceutically acceptable salt thereof, useful for the treatment of psoriasis.

Also disclosed herein are compounds and controlled release pharmaceutical compositions having particular pharmacokinetic characteristics, e.g., Cₘₐₓ, Tₘₐₓ, absorption lag time (T_{lag}), t_{1/2}, apparent oral clearance (CL/F; for parent compound only), apparent volume of distribution (V_{d}/F; for parent compound only), area under the concentration-time curve from time zero to the last quantifiable time interval (AUCₗₐₛₜ), and AUC_{infinity}.

### Controlled Release Pharmaceutical Compositions

The pharmaceutical compositions of the present invention comprise 2-(2,5-dioxopyrrolidin-1-yl)ethyl methyl fumarate, or a pharmaceutically acceptable salt thereof.

Also disclosed herein are pharmaceutical compositions comprising a controlled release polymer, and a compound of Formula (I): 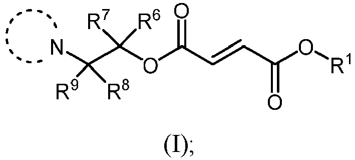 or a pharmaceutically acceptable salt thereof,
wherein:
R¹ is C₁-C₆ alkyl; 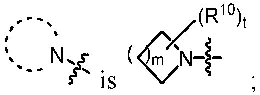
m is 1 or 2;
t is 0, 1, 2, 3, 4, 5, or 6;
R⁶, R⁷, R⁸ and R⁹ are each, independently, H, C₁-C₆ alkyl, or C(O)OR^{a};
R^{a} is H or C₁-C₆ alkyl; and
each R¹⁰ is, independently, H, halogen, C₁-C₆ alkyl, C₃-C₁₀ carbocycle, heterocycle comprising one or two 5- or 6-member rings and 1-4 heteroatoms selected from N, O and S, or heteroaryl comprising one or two 5- or 6-member rings and 1-4 heteroatoms selected from N, O and S;
or, alternatively, two R¹⁰s attached to the same carbon atom, together with the carbon atom to which they are attached, form a carbonyl;
or, alternatively, two R¹⁰s attached to adjacent atoms, together with the carbon atoms to which they are attached, form a fused C₃₋₆ ring.

In one embodiment, the pharmaceutical compositions provided herein comprise a compound of Formula (I), wherein R¹ is methyl.

In another embodiment, the pharmaceutical compositions provided herein comprise a compound of Formula (I), wherein R⁶, R⁷, R⁸ and R⁹ are each H.

In another embodiment, the pharmaceutical compositions provided herein comprise a compound of Formula (I), wherein m is 2.

In another embodiment, the pharmaceutical compositions provided herein comprise a compound of Formula (I), wherein t is 4, 5 or 6 and four R¹⁰s (where two pairs of R¹⁰s are each attached to the same carbon atoms, together with the carbon atoms to which they are attached) form two carbonyls.

In another embodiment, the pharmaceutical compositions provided herein comprise a compound of Formula (I), wherein R¹ is methyl and t is 4, 5 or 6 and four R¹⁰s (where two pairs of R¹⁰s are each attached to the same carbon atoms, together with the carbon atoms to which they are attached) form two carbonyls.

In another embodiment, the pharmaceutical compositions provided herein comprise a compound of Formula (I), wherein R⁶, R⁷, R⁸ and R⁹ are each H, t is 4, 5 or 6 and four R¹⁰s (where two pairs of R¹⁰s are each attached to the same carbon atoms, together with the carbon atoms to which they are attached) form two carbonyls.

In another embodiment, the pharmaceutical compositions provided herein comprise a compound of Formula (I), wherein m is 2, t is 4, 5 or 6 and four R¹⁰s (where two pairs of R¹⁰s are each attached to the same carbon atoms, together with the carbon atoms to which they are attached) form two carbonyls.

In another embodiment, the pharmaceutical compositions provided herein comprise a compound of Formula (I), wherein m is 2 and R¹ is methyl.

In another embodiment, the pharmaceutical compositions provided herein comprise a compound of Formula (I), wherein m is 2 and R⁶, R⁷, R⁸ and R⁹ are each H.

In another embodiment, the pharmaceutical compositions provided herein comprise a compound of Formula (I), wherein R¹ is methyl and R⁶, R⁷, R⁸ and R⁹ are each H.

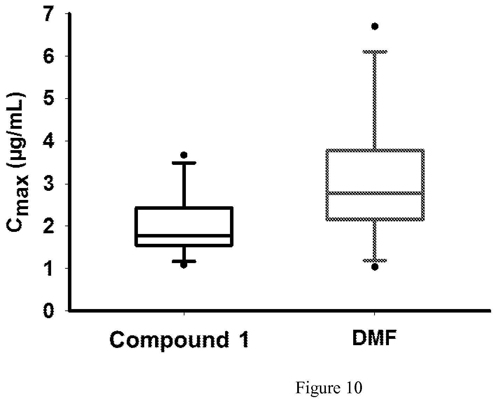

The pharmaceutical compositions provided herein can provide many therapeutic benefits that are not achieved with previously known fumarate ester preparations, such as dimethyl fumarate compositions or the currently marketed formulation, TECFIDERA®. For example, one benefit of the pharmaceutical compositions disclosed herein is that they can maintain lower, steadier plasma peak values, for example, Cₘₐₓ (see Table 4.1 and Figure 10), so as to reduce the incidence and severity of possible side effects (see Table 4.2). As another benefit, the pharmaceutical compositions disclosed herein can have a longer lag time for absorption, T_{lag} (see Table 4.1), so as to increase the period between administration of the pharmaceutical composition and detectable plasma levels of the drug.

In one embodiment, the pharmaceutical formulation comprises about 50% to about 90% 2-(2,5-dioxopyrrolidin-1-yl)ethyl methyl fumarate, by weight. In another embodiment, the pharmaceutical composition comprises about 70% to about 80% 2-(2,5-dioxopyrrolidin-1-yl)ethyl methyl fumarate, by weight.

In one embodiment, the pharmaceutical composition is in the form of a tablet or a plurality of minitablets. The minitablets are typically controlled or delayed-release minitablets. Controlled release may be achieved for example by applying an enteric polymer coating over the minitablet cores. For example, minitablets may have a diameter of about 2 mm, a thickness of about 2 mm and an enteric polymer coating thickness of from about 40 to about 60 microns.. These minitablets may be filled into capsules (such as gelatin or hydroxypropylmethyl cellulose (HPMC) capsules) to produce a final dosage form.

The pharmaceutical composition contains approximately 210 mg, 420 mg, 630 mg, 840 mg, or 980 mg of 2-(2,5-dioxopyrrolidin-1-yl)ethyl methyl fumarate. In another embodiment, the pharmaceutical composition contains approximately 420 mg of 2-(2,5-dioxopyrrolidin-1-yl)ethyl methyl fumarate.

In one specific embodiment, provided herein is a pharmaceutical composition comprising 2-(2,5-dioxopyrrolidin-1-yl)ethyl methyl fumarate, or a pharmaceutically acceptable salt thereof, microcrystalline cellulose, and methacrylic acid copolymer - Type C. In an embodiment, the pharmaceutical composition further comprises crospovidone or magnesium stearate. In another embodiment, the pharmaceutical composition comprises microcrystalline cellulose, crospovidone, magnesium stearate, and methacrylic acid copolymer - Type C. In another embodiment, the pharmaceutical composition comprises microcrystalline cellulose, crospovidone, magnesium stearate, methacrylic acid copolymer - Type C, talc, colloidal silicon dioxide, and triethyl citrate. In an embodiment, the pharmaceutical composition comprises about 50% to about 90% 2-(2,5-dioxopyrrolidin-1-yl)ethyl methyl fumarate, by weight. In yet another embodiment, the pharmaceutical composition comprises about 70% to about 80% 2-(2,5-dioxopyrrolidin-1-yl)ethyl methyl fumarate, by weight.

The pharmaceutical compositions provided herein can be administered orally, using a convenient daily dosage regimen that can be adjusted according to the degree of severity of the disease-state to be treated.

In another embodiment, a compound of Formula (I), e.g., 2-(2,5-dioxopyrrolidin-1-yl)ethyl methyl fumarate, is efficiently converted to the active species, i.e., monomethyl fumarate, upon oral administration.

For a drug to achieve its therapeutic effect, it is necessary to provide an appropriate level of blood or plasma concentration. Many drugs, including dimethyl fumarate, must be administered multiple times a day to maintain the required concentration. Furthermore, even with multiple administrations of such a drug per day, the blood or plasma concentrations of the active ingredient may still vary with time, i.e., at certain time points between administrations there are higher concentrations of the active ingredient than at other times. Thus, at certain time points of a 24-hour period, a patient may receive therapeutically effective amounts of the active ingredient, while at other time points the concentration of the active ingredient in the blood may fall below therapeutic levels. The present invention provides controlled or delayed-release compositions as described below.

In one embodiment, the pharmaceutical composition is a delayed-release or extended-release composition comprising a compound of Formula (I), and one or more pharmaceutically acceptable carriers, wherein the pharmaceutical composition provides a therapeutically effective amount of monomethyl fumarate to a subject. In another embodiment, the pharmaceutical composition provides a therapeutically effective amount of monomethyl fumarate to a subject for at least about 8 hours to at least about 24 hours. In another embodiment, the pharmaceutical composition provides a therapeutically effective amount of monomethyl fumarate to a subject for at least about 8 hours, at least about 10 hours, at least about 12 hours, at least about 13 hours, at least about 14 hours, at least about 15 hours, at least about 16 hours, at least about 17 hours, at least about 18 hours, at least about 19 hours, at least about 20 hours, at least about 21 hours, at least about 22 hours, at least about 23 hours, or at least about 24 hours or longer. For example, at least about 18 hours. For example, at least about 12 hours. For example, about 12 hours. For example, greater than 12 hours. For example, at least about 16 hours. For example, at least about 20 hours. For example, at least about 24 hours.

As used herein, the term "delayed-release" refers to a medication that does not immediately disintegrate and release the active ingredient(s) into the body. The term "delayed-release" is used with reference to a drug composition having a release profile in which there is a predetermined delay in the release of the drug following administration. In some embodiments, the delayed-release composition includes an enteric coating, which is a barrier applied to oral medication that prevents release of medication before it reaches the small intestine. Delayed-release compositions, such as enteric coatings, prevent drugs having an irritant effect on the stomach from dissolving in the stomach. Such coatings are also used to protect acid-unstable drugs from the stomach's acidic exposure, delivering them instead to the more basic pH environment of the intestine where they do not degrade, and give their desired action.

The controlled release composition of the invention may be in the form of tablets or mini-tablets (typically about 1mm to about 6mm in diameter depending on the shape of the mini-tablets and the size of capsule used to hold them). Tablet cores may be manufactured by blending the MMF prodrug with suitable excipients (such as glidants, diluents, binders, disintegrants, lubricants and the like) prior to tableting in a tablet press equipped with tooling adapted to produce tablets, or mini-tablets, of the desired size and shape. The controlled release characteristics of the tablets or mini-tablets may be tailored by the choice of excipients used to form the tablet cores and / or by applying a controlled release coating to the tablet cores. Mini-tablets may be filed into an appropriately sized capsule to produce a final dosage form.

Where a diluent is used it is preferably selected from the group consisting of microcrystalline cellulose, dextrose, lactose, sucrose, mannitol, dicalcium phosphate and combinations or mixtures thereof.

Where a disintegrant is used it is preferably selected from the group consisting of sodium carboxymethylcellulose, starch, crosslinked polyvinylpyrrolidone (crospovidone) and combinations or mixtures thereof,
Alternatively the controlled release composition of the invention may take the form of an extrudate. Typically the extrudate will be in the form of pellets which may be subjected to a spheronization process to produce spheres. These extrudate cores may be subjected to further processing, such as coating, prior to being filled into an appropriately sized capsule to produce a final dosage form. The controlled release characteristics of the extruded spheres may be tailored by the choice of excipients used during the extrusion process and / or by applying a controlled release coating to the extrudate cores.

In one embodiment a controlled release composition comprises delayed release extruded spheres or mini-tablets which are coated with an enteric coating. The enteric coating protects the drug substance from the low pH environment of the stomach, whilst providing rapid release once the mini-tablets exit the stomach and enter the higher pH environment of the small intestine. As will be appreciated by the person skilled in the art the coating level may be characterised in various ways terms such as percentage weight gain (% wt); weight of coating material added per unit area (mg / cm²) or in terms of coating thickness (µm). From a manufacturing perspective coating to a specified % weight gain is more practical as this can be more readily determined and controlled in-process. However, the other parameters, weight of coating added per unit area and coating thickness, also have advantages. Any amount of polymer may be applied to the extruded spheres or mini-tabs provided that it is sufficient to impart the abovementioned protection and rapid release characteristics as the spheres / mini-tablets pass along the gastrointestinal tract.

The controlled release polymer is preferably an enteric polymer, such as for example cellulose acetate phthalate, polyvinyl acetate phthalate, and acrylic acid and acrylate polymers and copolymers. Coating excipients such as plasticizers and anti-tack agents (sometimes also referred to as anti-adherrants) may be used to improve the coating process and the release and durability characteristics of the coating.

Wherein a plasticizer is used it is preferably selected from the group consisting of triacetin, tributyl citrate, triethyl citrate, dibutyl sebacate, diethyl phthalate and combinations or mixtures thereof.

Where an anti-tack agent is used it is preferably selected from the group consisting of colloidal silicon dioxide, talc and combinations or mixtures thereof.

For delayed release mini-tabs or extruded spheres the level of coating applied, characterised in terms of % weight gain (i.e., the % gain in weight of the mini-tab / sphere following application of the coating), may suitably be about 2% to about 30% weight gain (i.e. dry weight of polymer and coating excipients (if any) added expressed as a percent of the weight of uncoated beads) and is preferably 2 % to 15 % weight gain.

The coating level may also be characterised in terms of coating thickness (mm or µm). However, as will be appreciated by the skilled person the uncoated mini-tablets (the mini-tab cores) are typically cylindrical in shape, with flat or curved ends, rather than spherical and coating materials will not be applied with perfect uniformity across the entire surface of the cores. Furthermore, extruded cores, whilst roughly spherical in shape, may also exhibit some surface non-uniformity that can result in variation and imperfections in the coating process. Therefore, coating thickness may vary somewhat across different areas of any given core and also from one core to the next. Any coating thickness which provides enteric protection of the mini-tablet / extruded sphere cores whilst also facilitating rapid release of the prodrug once the low pH environment of the stomach is exited will suffice for the purposes of delayed release mini-tablets or extruded spheres. Preferably a delayed release coating will comprise a methacrylic acid copolymer and will have a thickness from about 40 to about 60 µm (microns).

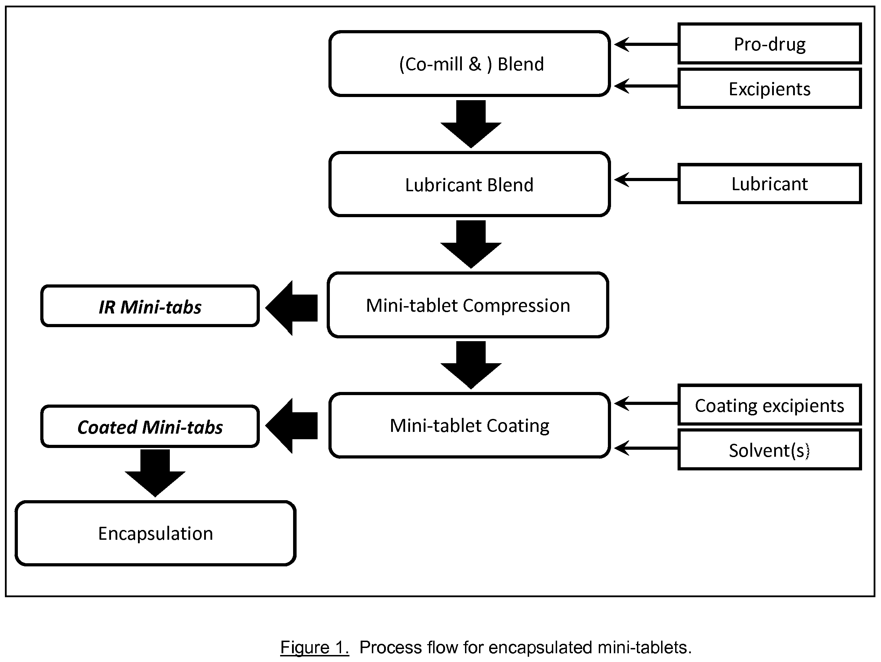

Figure 1 is a schematic representation of the process flow involved in a process for the manufacture of encapsulated coated mini-tablets according to the invention. Further details of the process are described below and in the Examples that follow. The resultant dosage form (capsules) deliver therapeutically efficacious levels of MMF whilst reducing gastric irritancy compared to other MMF-prodrug compositions. With reference to Figure 1:
∘ the MMF-prodrug is blended, and optionally co-milled, with tableting excipients (other than lubricant), in an intermediate bulk container, V-blender or the like;
∘ a lubricant is added and the mixture is blended for a further period;
∘ the resultant blend is fed into a tablet press where it is compressed into mini-tablets;
∘ the mini-tablet cores thus produced may be used as immediate release tablets or may be processed further (by the application of one or more functional coatings, to produce modified mini-tabs) in which case the next coating step applies;
∘ the mini-tablets are fed into a tablet coater in which a coating comprising coating excipients and solvent(s) - either as a coating solution or as a dispersion - is applied;
∘ the mini-tablets are filled into capsules up to the desired dosage strength using an encapsulator.

The type and number of coatings applied will determine the release characteristics of modified release mini-tablets. For example an enteric coating may be applied to provide for a delayed release such that the prodrug is release in a burst fashion after the mini-tablets have passed through the stomach. Alternatively extended release may be provided by applied an extended release coating underneath an enteric coating, so that the drug is released in an extended, continuous controlled fashion once the mini-tablets have exited the stomach. Different type of mini-tablets may be combined in a single capsule to produce a desired release profile. For example a combination of delayed release and extended release mini-tablets may be employed. Alternatively, immediate release and modified release mini-tabs may be mixed in different proportions and filled into capsules to provide dosage forms having different strengths and release profiles.

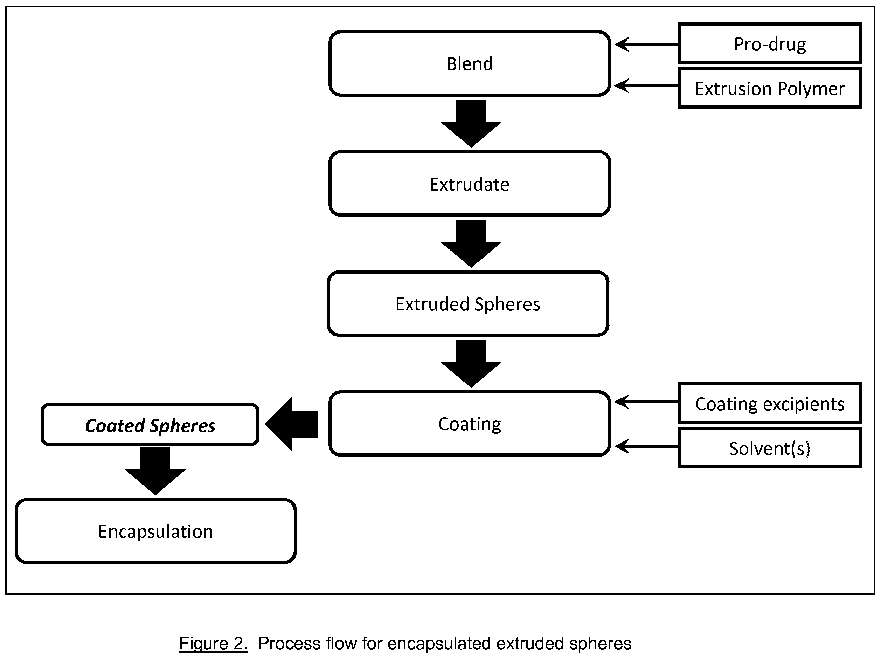

Figure 2 is a schematic representation of the process flow involved in a process for the manufacture of encapsulated coated extruded spheres according to the invention. Further details of the process are described below and in the Examples that follow. The resultant dosage form (capsules) deliver therapeutically efficacious levels of MMF. It is also believed that the coated extruded spheres of the invention may reduce gastric irritancy and other side effects often associated with MMF prodrug treatments. With reference to Figure 2:
∘ the MMF-prodrug is blended with the extrusion polymer, and any other excipients that may be required, in an intermediate bulk container, V-blender or the like;
∘ the resultant blend is fed into an extruder where it undergoes heating and is subsequently propelled through a die to produce extrudate strands (these strands will be cylindrical where a round die is utilised);
∘ the extrudate strands are chopped into smaller cylindrical or rod-like pellets which are rounded off into spheres;
∘ the extrudate spheres are then fed into a coater in which a coating comprising coating excipients and solvent(s) - either as a coating solution or as a dispersion - is applied;
∘ the coated extrudate spheres are filled into capsules up to the desired dosage strength using an encapsulator.

The type and number of coatings applied will determine the release characteristics of the final dosage form. For example an enteric coating may be applied to provide for a delayed release such that the prodrug is released in a burst fashion after the extruded spheres have passed through the stomach. Alternatively extended release may be provided by applied an extended release coating underneath an enteric coating, so that the drug is released in an extended, continuous controlled fashion once the extruded spheres have exited the stomach. Different types of spheres may be combined in a single capsule to produce a desired release profile. For example a combination of delayed release and extended release spheres may be employed. Alternatively, immediate release and modified release extruded spheres may be mixed in different proportions and filled into capsules to provide dosage forms having different strengths and release profiles.

The person skilled in the art will appreciate that in deciding on the choice of tableting and coating excipients key considerations are (i) compatability between the excipient(s) in question and the prodrug; and (ii) the potential for the excipient to impact on the release of prodrug from the composition. The former (excipient / prodrug compatability) may be readily determined by a compatability study, which is routine practice in the field of pharmaceutics. Whilst the latter (impact on prodrug release) may be readily determined by *in vitro* dissolution testing, which again is routine practice. Accordingly, the skilled person will readily understand that various permutations and combinations of excipients, described in general terms above, may be utilized provided that acceptable compatability and release characteristics are achieved.

### Compounds

The pharmaceutical compositions of the present invention comprise 2-(2,5-dioxopyrrolidin-1-yl)ethyl methyl fumarate, or a pharmaceutically acceptable salt thereof.

Also dislosed herein are pharmaceutical compositions comprising a compound of Formula (I), or a pharmaceutically acceptable salt thereof and an enteric polymer. In an embodiment, the pharmaceutical composition further comprises a disintegrant or lubricant. In another embodiment, the pharmaceutical composition comprises a disintegrant, a lubricant, and an enteric polymer.

In one embodiment, the pharmaceutical composition comprises a compound of Formula (I): 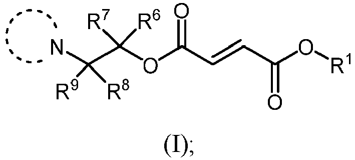 or a pharmaceutically acceptable salt thereof,
wherein R¹, R⁶, R⁷, R⁸, and R⁹ are defined as above.

In one embodiment, the pharmaceutical composition comprises a compound listed in Table A herein.

**Table A**

| | |
|---|---|
| 1 | 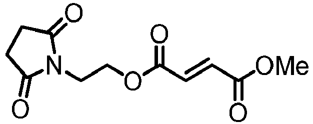 |
| 2 | 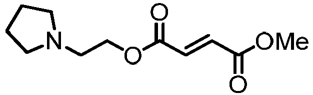 |
| 3 | 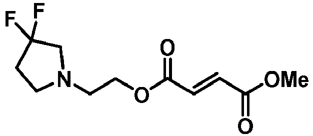 |
| 4 | 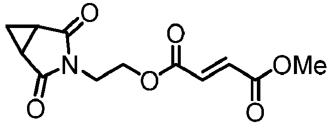 |
| 5 | 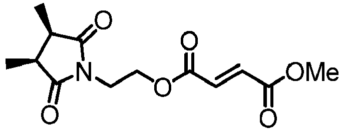 |
| 6 | 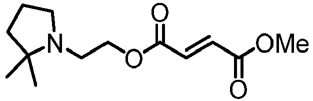 |
| 7 | 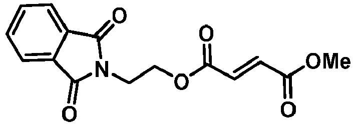 |
| 8 | 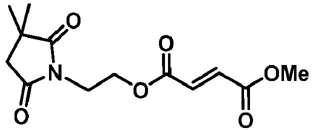 |
| 9 | 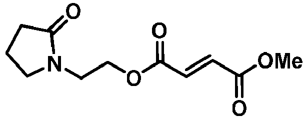 |
| 10 | 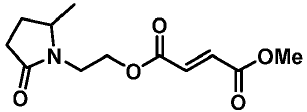 |
| 11 | 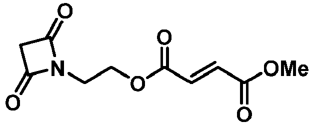 |
| 12 | 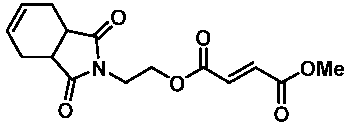 |

In another embodiment, a compound of Formula (I) is efficiently converted to the active species, *i.e.,* monomethyl fumarate, upon oral administration. For example, about 50 mole percent, about 55 mole percent, about 60 mole percent, about 65 mole percent, about 70 mole percent, about 75 mole percent, about 80 mole percent, about 85 mole percent, about 90 mole percent, or greater than 90 mole percent of the total dose of a compound of Formula (I) administered is converted to monomethyl fumarate upon oral administration. In another embodiment, any one of Compounds 1-12 is efficiently converted to the active species, *i.e.,* monomethyl fumarate, upon oral administration. For example, about 50 percent, about 55 percent, about 60 percent, about 65 percent, about 70 percent, about 75 percent, about 80 percent, about 85 percent, about 90 percent, or greater than 90 percent of the total dose of any one of Compounds 1-12 administered is converted to monomethyl fumarate upon oral administration.

As used herein, "alkyl", "C₁, C₂, C₃, C₄, C₅ or C₆ alkyl" or "C₁-C₆ alkyl" is intended to include C₁, C₂, C₃, C₄, C₅ or C₆ straight chain (linear) saturated aliphatic hydrocarbon groups and C₃, C₄, C₅ or C₆ branched saturated aliphatic hydrocarbon groups. For example, C₁-C₆ alkyl is intended to include C₁, C₂, C₃, C₄, C₅ and C₆ alkyl groups. Examples of alkyl include, moieties having from one to six carbon atoms, such as, but not limited to, methyl, ethyl, n-propyl, i-propyl, n-butyl, s-butyl, t-butyl, n-pentyl, s-pentyl, or n-hexyl.

In certain embodiments, a straight chain or branched alkyl has six or fewer carbon atoms (*e.g.,* C₁-C₆ for straight chain, C₃-C₆ for branched chain), and in another embodiment, a straight chain or branched alkyl has four or fewer carbon atoms.

"Aryl" includes groups with aromaticity, including "conjugated", or multicyclic, systems with at least one aromatic ring. Examples include phenyl, benzyl, naphthyl, etc.

"Heteroaryl" groups are aryl groups, as defined above, having from one to four heteroatoms in the ring structure, and may also be referred to as "aryl heterocycles" or "heteroaromatics". As used herein, the term "heteroaryl" is intended to include a stable 5-, 6-, or 7-membered monocyclic or 7-, 8-, 9-, 10-, 11- or 12-membered bicyclic aromatic heterocyclic ring which consists of carbon atoms and one or more heteroatoms, *e.g.*, 1 or 1-2 or 1-3 or 1-4 or 1-5 or 1-6 heteroatoms, independently selected from the group consisting of nitrogen, oxygen and sulfur. The nitrogen atom may be substituted or unsubstituted (*i.e.,* N or NR wherein R is H or other substituents, as defined). The nitrogen and sulfur heteroatoms may optionally be oxidized (*i.e.,* N→O and S(O)ₚ, where p=1 or 2). It is to be noted that total number of S and O atoms in the heteroaryl is not more than 1.

Examples of heteroaryl groups include pyrrole, furan, thiophene, thiazole, isothiazole, imidazole, triazole, tetrazole, pyrazole, oxazole, isoxazole, pyridine, pyrazine, pyridazine, pyrimidine, and the like.

As used herein, "Py" refers to pyridinyl.

Furthermore, the terms "aryl" and "heteroaryl" include multicyclic aryl and heteroaryl groups, *e.g.,* tricyclic, bicyclic, *e.g.,* naphthalene, benzoxazole, benzodioxazole, benzothiazole, benzoimidazole, benzothiophene, methylenedioxyphenyl, quinoline, isoquinoline, naphthrydine, indole, benzofuran, purine, benzofuran, deazapurine, or indolizine.

In the case of multicyclic aromatic rings, only one of the rings needs to be aromatic (*e.g.,* 2,3-dihydroindole), although all of the rings may be aromatic (*e.g.,* quinoline). The second ring can also be fused or bridged.

The aryl or heteroaryl aromatic ring can be substituted at one or more ring positions with such substituents as described above, for example, alkyl, alkenyl, akynyl, halogen, hydroxyl, alkoxy, alkylcarbonyloxy, arylcarbonyloxy, alkoxycarbonyloxy, aryloxycarbonyloxy, carboxylate, alkylcarbonyl, alkylaminocarbonyl, aralkylaminocarbonyl, alkenylaminocarbonyl, alkylcarbonyl, arylcarbonyl, aralkylcarbonyl, alkenylcarbonyl, alkoxycarbonyl, aminocarbonyl, alkylthiocarbonyl, phosphate, phosphonato, phosphinato, amino (including alkylamino, dialkylamino, arylamino, diarylamino, and alkylarylamino), acylamino (including alkylcarbonylamino, arylcarbonylamino, carbamoyl, and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Aryl groups can also be fused or bridged with alicyclic or heterocyclic rings, which are not aromatic so as to form a multicyclic system (*e.g*., tetralin, methylenedioxyphenyl).

As used herein, "carbocycle" or "carbocyclic ring" is intended to include any stable monocyclic, bicyclic or tricyclic ring having the specified number of carbons, any of which may be saturated, unsaturated, or aromatic. For example, a C₃-C₁₄ carbocycle is intended to include a monocyclic, bicyclic or tricyclic ring having 3, 4, 5, 6, 7, 8, 9, 10, 11, 12, 13 or 14 carbon atoms. Examples of carbocycles include, but are not limited to, cyclopropyl, cyclobutyl, cyclobutenyl, cyclopentyl, cyclopentenyl, cyclohexyl, cycloheptenyl, cycloheptyl, cycloheptenyl, adamantyl, cyclooctyl, cyclooctenyl, cyclooctadienyl, fluorenyl, phenyl, naphthyl, indanyl, adamantyl, and tetrahydronaphthyl. Bridged rings are also included in the definition of carbocycle, including, for example, [3.3.0]bicyclooctane, [4.3.0]bicyclononane, [4.4.0]bicyclodecane and [2.2.2]bicyclooctane. A bridged ring occurs when one or more carbon atoms link two non-adjacent carbon atoms. In one embodiment, bridge rings are one or two carbon atoms. It is noted that a bridge always converts a monocyclic ring into a tricyclic ring. When a ring is bridged, the substituents recited for the ring may also be present on the bridge. Fused (e.g., naphthyl, tetrahydronaphthyl) and spiro rings are also included.

As used herein, "heterocycle" includes any ring structure (saturated or partially unsaturated) which contains at least one ring heteroatom (e.g., N, O or S). Examples of heterocycles include, but are not limited to, morpholine, pyrrolidine, tetrahydrothiophene, piperidine, piperazine, and tetrahydrofuran.

Examples of heterocyclic groups include, but are not limited to, acridinyl, azocinyl, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolinyl, 2H,6H-1,5,2-dithiazinyl, dihydrofuro[2,3-b]tetrahydrofuran, furanyl, furazanyl, imidazolidinyl, imidazolinyl, imidazolyl, 1H-indazolyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isoxazolyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2,4-oxadiazol5(4H)-one, oxazolidinyl, oxazolyl, oxindolyl, pyrimidinyl, phenanthridinyl, phenanthrolinyl, phenazinyl, phenothiazinyl, phenoxathinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl.

The description of the disclosure herein should be construed in congruity with the laws and principals of chemical bonding. For example, it may be necessary to remove a hydrogen atom in order accommodate a substituent at any given location. Furthermore, it is to be understood that definitions of the variables (*i.e.,* "R groups"), as well as the bond locations of Formula (I) of the invention, will be consistent with the laws of chemical bonding known in the art. It is also to be understood that all of the compounds of the invention described above will further include bonds between adjacent atoms and/or hydrogens as required to satisfy the valence of each atom. That is, bonds and/or hydrogen atoms are added to provide the following number of total bonds to each of the following types of atoms: carbon: four bonds; nitrogen: three bonds; oxygen: two bonds; and sulfur: two-six bonds.

Compounds of the present invention can be prepared in a variety of ways using commercially available starting materials, compounds known in the literature, or from readily prepared intermediates, by employing standard synthetic methods and procedures either known to those skilled in the art, or which will be apparent to the skilled artisan in light of the teachings herein. Standard synthetic methods and procedures for the preparation of organic molecules and functional group transformations and manipulations can be obtained from the relevant scientific literature or from standard textbooks in the field. Although not limited to any one or several sources, classic texts such as Smith, M. B., March, J., March's Advanced Organic Chemistry Reactions, Mechanisms, and Structure, 5th edition, John Wiley & Sons: New York, 2001; and Greene, T. W., Wuts, P. G. M., Protective Groups in Organic Synthesis, 3rd edition, John Wiley & Sons: New York, 1999, are useful and recognized reference textbooks of organic synthesis known to those in the art. The descriptions of synthetic methods described herein are designed to illustrate, but not to limit, general procedures for the preparation of compounds of the present invention.

### Pharmacokinetics

In an embodiment, the pharmaceutical compositions display certain desirable pharmacokinetic characteristics. The plasma concentration of the active ingredient or drug at the point of maximum concentration, Cₘₐₓ, may be related to short-term side effects, or adverse events, which may follow administration of a dose of a drug, such as dimethyl fumarate. Typically upon administration of a drug the subject experiences a rapid spike in the plasma concentration of the drug. A high Cₘₐₓ can manifest in the subject as one or more adverse events. Thus, in order to reduce the probability of such adverse events, it can be desirable that a drug produces a lower Cₘₐₓ. Further, extending the absorption time (T_{lag}) of a drug can also be desirable as this presents a longer exposure of drug in the subject, which may provide a more effective treatment as compared to punctuated plasma concentrations of a drug.

The pharmaceutical compositions provided herein can provide many therapeutic benefits that are not achieved with previously known fumarate ester preparations, such as dimethyl fumarate compositions or the currently marketed formulation, TECFIDERA®. For example, one benefit of the pharmaceutical compositions disclosed herein is that they can maintain a longer lag time for absorption, T_{lag} (see Table 4.1), so as to increase the period between administration of the pharmaceutical composition and detectable plasma levels of the drug. As another benefit, the pharmaceutical compositions disclosed herein can have lower, steadier plasma peak values, for example, Cₘₐₓ (see Table 4.1 and Figure 10), so as to reduce the incidence and severity of possible side effects (see Table 4.2).

Dislcosed herein is a pharmaceutical composition comprising a compound of Formula (I) for use in a method of treating multiple sclerosis, comprising administering to a subject in need thereof said pharmaceutical composition: 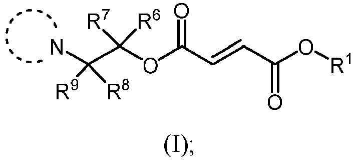 or a pharmaceutically acceptable salt thereof,
wherein R¹, R⁶, R⁷, R⁸, and R⁹ are defined as above; and
further wherein administering the pharmaceutical composition provides one or more of the following pharmacokinetic parameters:
i) a mean monomethyl fumarate Cₘₐₓ of from about 1.8 µg/mL to about 2.5 µg/mL in the plasma of the subject;
ii) a mean monomethyl fumarate AUCₗₐₛₜ of from about 4.0 µg·hr/mL to about 5.0 µg·hr/mL in the plasma of the subject;
iii) a median monomethyl fumarate Tₘₐₓ of from about 2.75 hours to about 3.5 hours in the plasma of the subject;
iv) a median monomethyl fumarate terminal elimination half-life (t_{1/2}) of from about 0.65 hours to about 0.8 hours in the plasma of the subject; and
v) a median monomethyl fumarate T_{lag} for absorption of from about 1 hour to about 2 hours.

In one embodiment, the pharmaceutical composition provides one or more of the following pharmacokinetic parameters:
i) a mean monomethyl fumarate Cₘₐₓ of about 2.0 µg/mL in the plasma of the subject;
ii) a mean monomethyl fumarate AUCₗₐₛₜ of about 4.2 µg·hr/mL in the plasma of the subject;
iii) a median monomethyl fumarate Tₘₐₓ of about 3 hours in the plasma of the subj ect;
iv) a median monomethyl fumarate terminal elimination half-life (t_{1/2}) of about 0.75 hours in the plasma of the subject; and
v) a median monomethyl fumarate T_{lag} for absorption of about 1.5 hours.

In one embodiment, the pharmaceutical composition provides the following pharmacokinetic parameters:
i) a mean monomethyl fumarate Cₘₐₓ of about 2.0 µg/mL in the plasma of the subject;
ii) a mean monomethyl fumarate AUCₗₐₛₜ of about 4.2 µg·hr/mL in the plasma of the subject; and
iii) a mean monomethyl fumarate T_{lag} for absorption of about 1.5 hours.

In one embodiment, the compound of Formula (I) is 2-(2,5-dioxopyrrolidin-1-yl)ethyl methyl fumarate.

In one embodiment, the pharmaceutical composition is in a solid oral dosage form. In another embodiment, the solid oral dosage form comprises approximately 210 mg to approximately 630 mg of 2-(2,5-dioxopyrrolidin-1-yl)ethyl methyl fumarate and further wherein administration to a subject provides one or more of the following pharmacokinetic parameters:
i) a mean monomethyl fumarate Cₘₐₓ of from about 1.8 µg/mL to about 2.5 µg/mL in the plasma of the subject;
ii) a mean monomethyl fumarate AUCₗₐₛₜ of from about 4.0 µg·hr/mL to about 5.0 µg·hr/mL in the plasma of the subject;
iii) a median monomethyl fumarate Tₘₐₓ of from about 2.75 hours to about 3.5 hours in the plasma of the subject;
iv) a median monomethyl fumarate terminal elimination half-life (t_{1/2}) of from about 0.65 hours to about 0.8 hours in the plasma of the subject; and
v) a median monomethyl fumarate T_{lag} for absorption of from about 1 hour to about 2 hours.

In a further embodiment, the solid oral dosage form contains approximately 420 mg of 2-(2,5-dioxopyrrolidin-1-yl)ethyl methyl fumarate.

The pharmaceutical compositions provided herein may also be characterized by their pharmacokinetic parameters. As used herein, "pharmacokinetic parameters" describe the in vivo characteristics of the active ingredient over time, including for example plasma concentration of the active ingredient.

As used herein, "Cₘₐₓ" means the measured plasma concentration of the active ingredient or drug (such as monomethyl fumarate (MMF)) at the point of maximum concentration.

As used herein, "Tₘₐₓ" refers to the time at which the plasma concentration of the active ingredient or drug (such as MMF) is the highest.

As used herein, "AUC" is the area under the curve of a graph of the concentration (typically plasma concentration) of the active ingredient or drug (such as MMF) vs. time, measured from one time to another.

As used herein, "T_{lag}" refers to the absorption lag time of the active ingredient.

As used herein, "t_{1/2}" refers to the half-life of the active ingredient or drug (such as MMF) in the subject (typically in the plasma).

As used herein, "Tₗₐₛₜ" refers to the final timepoint for which data was recorded.

As used herein, "%CV" refers to the coefficient of variance between subjects.

### Methods of Treatment

A neurological disease is a disorder of the brain, spinal cord or nerves in a subject. In one embodiment, the neurological disease is characterized by demyelination, or degeneration of the myelin sheath, of the central nervous system. The myelin sheath facilitates the transmission of nerve impulses through a nerve fiber or axon. In another embodiment, the neurological disease is selected from the group consisting of multiple sclerosis (MS), Alzheimer's disease, cerebral palsy, spinal cord injury, Amyotrophic lateral sclerosis (ALS), stroke, Huntington's disease, Parkinson's disease, optic neuritis, Devic disease, transverse myelitis, acute disseminated encephalomyelitis, adrenoleukodystrophy and adrenomyeloneuropathy, acute inflammatory demyelinating polyneuropathy (AIDP), chronic inflammatory demyelinating polyneuropathy (CIDP), acute transverse myelitis, progressive multifocal leucoencephalopathy (PML), acute disseminated encephalomyelitis (ADEM), and other hereditary disorders, such as leukodystrophies, Leber's optic atrophy, and Charcot-Marie-Tooth disease. In some embodiments, the neurological disorder is an auto-immune disease. In one embodiment, the neurological disease is multiple sclerosis. In another embodiment, the neurological disease is stroke. In another embodiment, the neurological disease is Alzheimer's disease. In another embodiment, the neurological disease is cerebral palsy. In another embodiment, the neurological disease is spinal cord injury. In another embodiment, the neurological disease is ALS. In another embodiment, the neurological disease is Huntington's disease. *See, e.g.,* US Patent No. 8,007,826, WO2005/099701 and WO2004/082684.

There are four major clinical types of MS: 1) relapsing-remitting MS (RRMS), characterized by clearly defined relapses with full recovery or with sequelae and residual deficit upon recovery; periods between disease relapses characterized by a lack of disease progression; 2) secondary progressive MS (SPMS), characterized by initial relapsing remitting course followed by progression with or without occasional relapses, minor remissions, and plateaus; 3) primary progressive MS (PPMS), characterized by disease progression from onset with occasional plateaus and temporary minor improvements allowed; and 4) progressive relapsing MS (PRMS), characterized by progressive disease onset, with clear acute relapses, with or without full recovery; periods between relapses characterized by continuing progression.

Clinically, the illness most often presents as a relapsing-remitting disease and, to a lesser extent, as steady progression of neurological disability. Relapsing-remitting MS (RRMS) presents in the form of recurrent attacks of focal or multifocal neurologic dysfunction. Attacks may occur, remit, and recur, seemingly randomly over many years. Remission is often incomplete and as one attack follows another, a stepwise downward progression ensues with increasing permanent neurological deficit. The usual course of RRMS is characterized by repeated relapses associated, for the majority of patients, with the eventual onset of disease progression. The subsequent course of the disease is unpredictable, although most patients with a relapsing-remitting disease will eventually develop secondary progressive disease. In the relapsing-remitting phase, relapses alternate with periods of clinical inactivity and may or may not be marked by sequelae depending on the presence of neurological deficits between episodes. Periods between relapses during the relapsing-remitting phase are clinically stable. On the other hand, patients with progressive MS exhibit a steady increase in deficits, as defined above and either from onset or after a period of episodes, but this designation does not preclude the further occurrence of new relapses.

Provided herein is a pharmaceutical composition described herein for use in a method of treating multiple sclerosis, comprising administering to a subject in need thereof a therapeutically effective amount ofsaid pharmaceutical composition.

Also disclosed herein is a pharmaceutical composition comprising a compound of Formula (I) for use in a method of treating a neurological disorder by administering to a subject in need thereof a therapeutically effective amount of a said pharmaceutical composition: 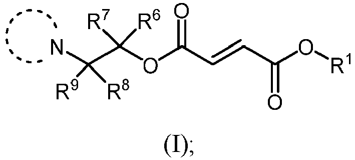 or a pharmaceutically acceptable salt thereof,
wherein R¹, R⁶, R⁷, R⁸, and R⁹ are defined as above.

In one embodiment the compound is 2-(2,5-dioxopyrrolidin-1-yl)ethyl methyl fumarate.

Also disclosed herein is a pharmaceutical composition comprising a compound of Formula (I) for use in a method of treating psoriasis by administering to a subject in need thereof a therapeutically effective amount of said pharmaceutical composition : 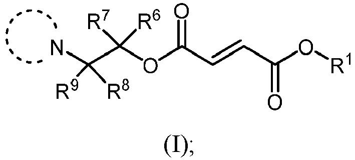 or a pharmaceutically acceptable salt thereof,
wherein R¹, R⁶, R⁷, R⁸, and R⁹ are defined as above.

In one embodiment the compound is 2-(2,5-dioxopyrrolidin-1-yl)ethyl methyl fumarate.

Also provided herein are compounds and pharmaceutical compositions described herein for use in methods of reducing the probability of an occurrence of a drug-induced gastrointestinal disorder in a subject who is currently being treated with dimethyl fumarate (such as TECFIDERA®) or who is contemplating treatment with dimethyl fumarate.

In one embodiment, provided herein is a pharmaceutical composition of the present invention for use in a method of reducing the probability of an occurrence of a drug-induced gastrointestinal disorder in a subject who is currently being treated with dimethyl fumarate (such as TECFIDERA®) or who is contemplating treatment with dimethyl fumarate. In another embodiment, provided herein is 2-(2,5-dioxopyrrolidin-1-yl)ethyl methyl fumarate for use in a method of reducing the probability of an occurrence of a drug-induced gastrointestinal disorder in a subject who is currently being treated with dimethyl fumarate (such as TECFIDERA®) or who is contemplating treatment with dimethyl fumarate.

In one ambodiment, the gastrointestinal disorder is selected from the group comprising diarrhea, eructation, flatulence, nausea, and retching. In another embodiment, the gastrointestinal disorder is nausea.

Also provided herein are compounds and pharmaceutical compositions described herein for use in methods of reducing the inter-patient variability of a monomethyl fumarate pharmacokinetic parameter in a patient population being treated with dimethyl fumarate (such as TECFIDERA®).

In one embodiment, provided herein is a compound described herein, or a pharmaceutically acceptable salt thereof, for use in a method of reducing the inter-patient variability of a monomethyl fumarate pharmacokinetic parameter in a patient population being treated with dimethyl fumarate.

In one embodiment, the monomethyl fumarate pharmacokinetic parameter is mean Cₘₐₓ, and further wherein a mean monomethyl fumarate Cₘₐₓ of about 2.0 µg/mL is achieved in the plasma of the subject with a %CV of less than 40%.

In one embodiment, the monomethyl fumarate pharmacokinetic parameter is mean AUCₗₐₛₜ, and further wherein a mean monomethyl fumarate AUCₗₐₛₜ of about 4.2 µg·hr/mL is achieved in the plasma of the subject with a %CV of less than 35%.

Also provided herein are compounds described herein, or a pharmaceutically acceptable salt thereof, for use in methods of treating a neurological disorder in a patient population, comprising administering to each patient a therapeutically effective amount of said compound .

In one embodiment, provided herein is a compound described herein, or a pharmaceutically acceptable salt thereof, for use in a method of treating a neurological disorder in a patient population, comprising administering to each patient a therapeutically effective amount of said compound;
wherein the inter-patient variability of a monomethyl fumarate pharmacokinetic parameter in the patient population is reduced relative to the patient population when treated with dimethyl fumarate.

In one embodiment, the monomethyl fumarate pharmacokinetic parameter is mean Cₘₐₓ, and further wherein a mean monomethyl fumarate Cₘₐₓ of about 2.0 µg/mL is achieved in the plasma of the subject with a %CV of less than 40%.

In one embodiment, the monomethyl fumarate pharmacokinetic parameter is mean AUCₗₐₛₜ, and further wherein a mean monomethyl fumarate AUCₗₐₛₜ of about 4.2 µg·hr/mL is achieved in the plasma of the subject with a %CV of less than 35%.

In one embodiment, the neurological disorder is multiple sclerosis or psoriasis.

Also provided herein are compounds described herein, or a pharmaceutically acceptable salt thereof, for use in methods of treating a neurological disorder in a subj ect.

In one embodiment, provided herein is a compound described herein, or a pharmaceutically acceptable salt thereof, for use in a method of treating a neurological disorder in a subject;
wherein one or more of the resultant monomethyl fumarate pharmacokinetic parameters exhibits reduced variability relative to a patient population treated with dimethyl fumarate.

In one embodiment, the monomethyl fumarate pharmacokinetic parameter is mean Cₘₐₓ, and further wherein a mean monomethyl fumarate Cₘₐₓ of about 2.0 µg/mL is achieved in the plasma of the subject with a %CV of less than 40%.

In one embodiment, the monomethyl fumarate pharmacokinetic parameter is mean AUCₗₐₛₜ, and further wherein a mean monomethyl fumarate AUCₗₐₛₜ of about 4.2 µg·hr/mL is achieved in the plasma of the subject with a %CV of less than 35%.

In one embodiment, the neurological disorder is multiple sclerosis or psoriasis.

As used herein, the terms "treating" or "treatment" of a disease, disorder, or syndrome, means inhibiting the disease, disorder, or syndrome, that is, arresting its development; and relieving the disease, disorder, or syndrome, that is, causing regression of the disease, disorder, or syndrome.

As used herein, the terms "effective amount" or "pharmaceutically effective amount" or "therapeutically effective amount" refer to a sufficient amount of an agent to provide the desired biological, therapeutic, and/or prophylactic result. That result can be reduction, amelioration, palliation, lessening, delaying, and/or alleviation of one or more of the signs, symptoms, or causes of a disease, or any other desired alteration of a biological system.

As used herein, a "subject in need thereof' is a subject having a neurological disease. In one embodiment, a subject in need thereof has multiple sclerosis. A "subject" includes a mammal. The mammal can be, *e.g.,* any mammal, *e.g.,* a human, primate, bird, mouse, rat, fowl, dog, cat, cow, horse, goat, camel, sheep or a pig. In one embodiment, the mammal is a human.

### EXPERIMENTAL

### Example 1 - Synthesis of Selected Compounds of Formula (I)

### General Procedure 1

To a mixture of monomethyl fumarate (MMF) (1.0 equivalent) and HBTU (1.5 equivalents) in dimethylformamide (25 ml per g of MMF) was added Hünigs base (2.0 equivalents). The dark brown solution was stirred for 10 minutes, where turned into a brown suspension, before addition of the alcohol (1.0 - 1.5 equivalents). The reaction was stirred for 18 hours at room temperature. Water was added and the product extracted into ethyl acetate three times. The combined organic layers were washed with water three times, dried with magnesium sulphate, filtered and concentrated in vacuo at 45 °C to give the crude product. The crude product was purified by silica chromatography and in some cases further purified by trituration with diethyl ether to give the clean desired ester product. All alcohols were either commercially available or made following known literature procedures.

As an alternative to HBTU (*N,N,N',N'*-Tetramethyl-*O*-(1*H*-benzotriazol-1-yl)uronium hexafluorophosphate), any one of the following coupling reagents can be used: EDCI/HOBt (*N*-(3-dimethylaminopropyl)-*N'*-ethylcarbodiimide hydrochloride/hydroxybenzotriazole hydrate); COMU ((1-cyano-2-ethoxy-2-oxoethylidenaminooxy)dimethylamino-morpholino-carbenium hexafluorophosphate); TBTU (*O*-(benzotriazol-1-yl)-*N,N,N',N'*-tetramethyluronium tetrafluoroborate); TATU (O-(7-azabenzotriazole-1-yl)-1,1,3,3-tetramethyluronium tetrafluoroborate); Oxyma (ethyl (hydroxyimino)cyanoacetate); PyBOP ((benzotriazol-1-yloxy)tripyrrolidinophosphonium hexafluorophosphate); HOTT (*S*-(1-oxido-2-pyridyl)-*N,N,N',N'*-tetramethylthiuronium hexafluorophosphate); FDPP (pentafluorophenyl diphenylphosphinate); T3P (propylphosphonic anhydride); DMTMM (4-(4,6-dimethoxy-1,3,5-triazin-2-yl)-4-methylmorpholinium tetrafluoroborate); PyOxim ([ethyl cyano(hydroxyimino)acetato-*O*²]tri-1-pyrrolidinylphosphonium hexafluorophosphate); TSTU (*N,N,N',N'*-tetramethyl-*O*-(*N-*succinimidyl)uronium tetrafluoroborate); TDBTU (*O*-(3,4-dihydro-4-oxo-1,2,3-benzotriazin-3-yl)-*N,N,N',N'*-tetramethyluronium tetrafluoroborate); TPTU (*O*-(2-oxo-1(2*H*)pyridyl)-*N,N,N',N'*-tetramethyluronium tetrafluoroborate); TOTU (*O-*[(ethoxycarbonyl)cyanomethylenamino]-*N,N,N',N'*-tetramethyluronium tetrafluoroborate); IIDQ (isobutyl 1,2-dihydro-2-isobutoxy-1-quinolinecarboxylate); or PyCIU (chlorodipyrrolidinocarbenium hexafluorophosphate),

As an alternative to Hünig's base (diisopropylethylamine), any one of the following amine bases can be used: triethylamine; tributylamine; triphenylamine; pyridine; lutidine (2,6-dimethylpyridine); collidine (2,4,6-trimethylpyridine); imidazole; DMAP (4-(dimethylamino)pyridine); DABCO (1,4-diazabicyclo[2.2.2]octane); DBU (1,8-diazabicyclo[5.4.0]undec-7-ene); DBN (1,5-diazabicyclo[4.3.0]non-5-ene); or PROTON SPONGE (*N,N,N',N'*-tetramethyl-1,8-naphthalenediamine).

### General Procedure 2 - Conversion of the Ester Product into the Hydrochloride Salt

To a mixture of the ester product in diethyl ether (25 ml per g) was added 2M HCl in diethyl ether (1.5 equivalents). The mixture was stirred at room temperature for two hours. The solvent was decanted, more diethyl ether added and the solvent decanted again. The remaining mixture was then concentrated in vacuo at 45 °C and further dried in a vacuum oven at 55 °C for 18 hours to give the solid HCl salt.

### General Procedure 3

To a 100 mL, one-necked, round-bottomed flask, fitted with a magnetic stirrer and nitrogen inlet/outlet, were added 11 mL of an MTBE solution containing freshly prepared mono-methyl fumaryl chloride (4.9 g, 33 mmol) and 50 mL of additional MTBE at 20 °C. The resulting yellow solution was cooled to <20 °C with an ice water bath. Then, the alcohol, (33 mmol, 1 eq) was added dropwise, via syringe, over approximately 10 minutes. The reaction mixture was allowed to stir at <20 °C for 10 minutes after which time the cooling bath was removed and the reaction was allowed to warm to 20 °C and stir at 20 °C temperature for 16 hours. The reaction was deemed complete by TLC after 16 hours at RT. The reaction mixture was filtered through a medium glass fritted funnel to collect the off-white solids. The solids were dried in a vacuum oven at 25 °C overnight to afford the final product as an HCl salt. All alcohols were either commercially available or made following known literature procedures.

### General Procedure 4 - Alkylation with an Appropriate Alkyl Chloride

A mixture of monomethyl fumarate (MMF) (1.3 equivalent), the alkyl chloride (1 equivalent), and potassium carbonate (1.5 equivalent) in acetonitrile or dimethylformamide (50 ml per g of MMF) was heated at 20 to 65 °C overnight. The mixture was partitioned between ethyl acetate and saturated aqueous sodium hydrogen carbonate, and the organic phase dried (MgSO₄). Filtration and removal of the solvent under reduced pressure gave the crude product which was further purified by silica chromatography.

### Chemical Analysis/Procedures

The NMR spectra described herein were obtained with a Varian 400 MHz NMR spectrometer using standard techniques known in the art.

### 2-(2,5-dioxopyrrolidin-1-yl)ethyl methyl fumarate (Compound 1)

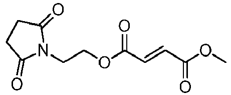

2-(2,5-dioxopyrrolidin-1-yl)ethyl methyl fumarate **1** was synthesized following general procedure 1 (1.03 g, 35 %).
¹H NMR (400 MHz, DMSO): δ 6.81 (2H, dd, J = 15.8 Hz); 4.36 (2H, t, J = 5.3 Hz); 3.84 (2H, t, J = 5.1 Hz); 3.80 (3H, s); 2.73 (4H, s). [M+H]⁺ = 256.07.

### Methyl (2-(pyrrolidin-1-yl)ethyl) fumarate hydrochloride (Compound 2)

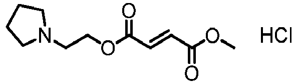

Methyl (2-(pyrrolidin-1-yl)ethyl) fumarate hydrochloride 2 was synthesized following general procedure 3.
¹H NMR (400 MHz, DMSO-d6) δ 11.12 (s, 1H), 6.94 (d, *J=* 15.8 Hz, 1H), 6.82 (d, *J* = 15.8 Hz, 1H), 4.53 - 4.46 (m, 2H), 3.76 (s, 3H), 3.61 - 3.45 (m, 4H), 3.11 - 2.94 (m, 2H), 2.06 - 1.79 (m, 4H). [M+H]⁺ = 228.46.

### 2-(3,3-difluoropyrrolidin-1-yl)ethyl methyl fumarate hydrochloride (Compound 3)

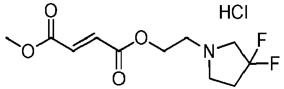

2-(3,3-Difluoropyrrolidin-1-yl)ethyl methyl fumarate **3** was synthesised from 2-(3,3-difluoropyrrolidin-1-yl)ethanol following general procedure 1.

2-(3,3-difluoropyrrolidin-1-yl)ethyl methyl fumarate was converted to 2-(3,3-difluoropyrrolidin-1-yl)ethyl methyl fumarate hydrochloride following general procedure 2 (0.55 g, 69 %).
¹H NMR (300 MHz, DMSO); δ 6.79 (2H, d); 4.20-4.39 (2H, m), 3.81 (2H, t), 3.66 (3H, s), 3.53-3.65 (4H, m), 2.54 (2H, sep). *m*/*z* [M+H]⁺ = 264.14.

### 2-(2,4-Dioxo-3-azabicyclo[3.1.0]hexan-3-yl)ethyl methyl fumarate (Compound 4)

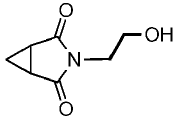

3-oxabicyclo[3.1.0]hexane-2,4-dione (1.0 g, 8.9 mmol) and ethanolamine (545 mg, 8.9 mmol) were heated neat at 200 °C for 2 hours. The crude reaction mixture was purified by silica chromatography (EtOAc) giving 3-(2-Hydroxyethyl)-3-azabicyclo[3.1.0]hexane-2,4-dione (1.06 g, 77%).
¹H NMR (300 MHz, CDCl₃): δ 3.71 (2H, t), 3.56 (2H, t), 2.51 (2H, dd), 1.95 (1H, br s), 1.59-1.43 (2H, m).

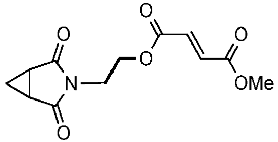

2-(2,4-dioxo-3-azabicyclo[3.1.0]hexan-3-yl)ethyl methyl fumarate **4** was synthesised from 3-(2-Hydroxyethyl)-3-azabicyclo[3.1.0]hexane-2,4-dione following general procedure 1 (452 mg, 53 %).
¹H NMR (300 MHz, CDCl₃): δ 6.81 (2H, d), 4.28 (2H, t), 3.80 (3H, s), 3.69 (2H, t), 2.48 (2H, dd), 1.59-1.49 (1H, m), 1.44-1.38 (1H, m). *m*/*z* [M+H]⁺ = 268.11.

### 2-((3R,4S)-3,4-Dimethyl-2,5-dioxopyrrolidin-1-yl)ethyl methyl fumarate (Compound 5)

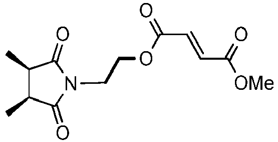

Racemic 2-((3R,4S)-3,4-dimethyl-2,5-dioxopyrrolidin-1-yl)ethyl methyl fumarate **5** was synthesised from racemic (3R,4S)-1-(2-hydroxyethyl)-3,4-dimethylpyrrolidine-2,5-dione following general procedure 1 (0.54 g, 44%).
¹H NMR (300 MHz, CDCl₃); 6.81-6.80 (2H, m), 4.37 (2H, t), 3.82 (2H, t), 3.80 (3H, s), 3.00-2.88 (2H, m), 1.25-1.18 (6H, m). *m*/*z* [M+H]⁺ = 284.2

### 2-(2,2-Dimethyl-5-oxopyrrolidin-1-yl)ethyl methyl fumarate (Compound 6)

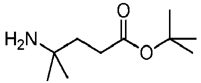

Tert-butyl acrylate (19.7 mL, 134.8 mmol) was added dropwise over 10 minutes to a refluxing solution of 2-nitropropane and Triton B (40% in methanol) (440 µL) in ethanol (50 mL). The reaction was heated at reflux overnight. The reaction solvent was removed under reduced pressure giving a crude residue that was dissolved in ethanol (200 mL) and hydrogenated overnight (300 psi) using Raney nickel (approximately 15 g). The reaction was filtered through celite. The solvent was removed under reduced pressure giving tert-butyl 4-amino-4-methylpentanoate (15.82 g, 63% yield).
¹H NMR (300 MHz, CDCl₃): δ 2.26 (2H, t), 1.65 (2H, t), 1.43 (9H, s), 1.68 (6H, s).

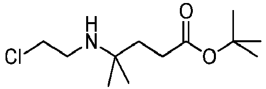

To a solution of tert-butyl 4-amino-4-methylpentanoate (3.0 g, 16.04 mmol) in methanol (100 mL) was added chloroacetaldehyde (45% in H₂O) (6.7 mL, 38.4 mmol) followed by acetic acid (2 mL, 35.0 mmol). After 1.5 hours sodium cyanoborohydride (1.51 g, 24.0 mmol) was added and the mixture stirred at room temperature for 3 hours. The reaction was partitioned between saturated aqueous sodium hydrogen carbonate (100 mL) and dichloromethane (300 mL). The organic phase was dried (MgSO₄). Filtration and removal of the solvent under reduced pressure gave tert-butyl 4-((2-chloroethyl)amino)-4-methylpentanoate (3.90 g, 98% yield).
¹H NMR (300 MHz, CDCl₃): δ 3.63 (2H, t), 2.85 (2H, t), 2.24 (2H, t), 1.67 (2H, t), 1,44 (9H, s), 1.07 (6H, s).

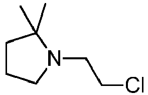

A mixture of tert-butyl 4-((2-chloroethyl)amino)-4-methylpentanoate (3.9 g, 15.7 mmol) and trifluoroacetic acid (27 mL) in dichloromethane (80 mL) were stirred at room temperature overnight. The reaction mixture was concentrated under reduced pressure. The residue was dissolved in further dichloromethane and concentrated again. This was repeated a further 3 times until the majority of the excess trifluoroacetic acid had been removed. The residue was dissolved in dichloromethane (500 mL) and N-(3-Dimethylaminopropyl)-N'-ethylcarbodiimide hydrochloride (4.61 g, 24.1 mmol), hydroxybenzotriazole hydrate (3.25 g, 24.1 mmol) and diisopropylethylamine (21 mL, 120 mmol) added. The mixture was stirred at room temperature overnight. The reaction was washed with water (300 mL) and dried (MgSO₄). Filtration and removal of the solvent under reduced pressure gave a crude residue that was purified by silica chromatography (heptane to ethyl acetate) giving 1-(2-chloroethyl)-5,5-dimethylpyrrolidin-2-one (1.24 g, 44% yield).
¹H NMR (300 MHz, CDCl3): δ 3.61 (2H, t), 3.41 (2H, t), 2.38 (2H, t), 1.88 (2H, t), 1.24 (6H, s).

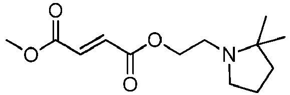

2-(2,2-Dimethyl-5-oxopyrrolidin-1-yl)ethyl methyl fumarate 6 was synthesised from 1-(2-chloroethyl)-5,5-dimethylpyrrolidin-2-one following general procedure 4 (1.02 g, 41 %).
¹H NMR (300 MHz, CDCl₃); 6.85 (2H, d), 4.33 (2H, t), 3.80 (3H, s), 3.41 (2H, t), 2.39 (2H, t), 1.88 (2H, t), 1.23 (6H, s). *m*/*z* [M+H]⁺ = 270.17.

### 2-(1,3-Dioxoisoindolin-2-yl)ethyl methyl fumarate (Compound 7)

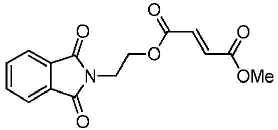

2-(1,3-Dioxoisoindolin-2-yl)ethyl methyl fumarate 7 was synthesised from 2-(2-hydroxyethyl)isoindoline-1,3-dione following general procedure 1 (0.63 g, 79%).
¹H NMR (300 MHz, MeOD); 7.87-7.77 (4H, m), 6.74-6.73 (2H, m), 4.45-4.40 (2H, m), 4.01-3.96 (2H, m), 3.76 (3H, s). *m*/*z* [M+H]⁺ = 304.1

### 2-(3,3-Dimethyl-2,5-dioxopyrrolidin-1-yl)ethyl methyl fumarate (Compound 8)

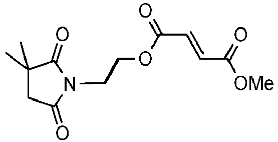

2-(3,3-Dimethyl-2,5-dioxopyrrolidin-1-yl)ethyl methyl fumarate 8 was synthesised from 1-(2-hydroxyethyl)-3,3-dimethylpyrrolidine-2,5-dione following general procedure 1 (0.72 g, 74%).
¹H NMR (300 MHz, CDCl₃); 6.83 (1H, d), 6.77 (1H, d), 4.38 (2H, t), 3.82 (1H, t), 3.80 (3H, s), 2.55 (2H, s), 1.31 (6H, s). *m*/*z* [M+H]⁺ = 284.1

### Methyl (2-(2-oxopyrrolidin-1-yl)ethyl) fumarate (Compound 9)

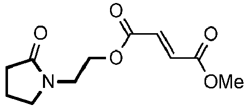

Methyl (2-(2-oxopyrrolidin-1-yl)ethyl) fumarate **9** was synthesised from 1-(2-hydroxyethyl)pyrrolidin-2-one following general procedure 1 (0.68 g, 73%).
¹H NMR (300 MHz, MeOD); 6.85 (2H, s), 4.33 (2H, t), 3.80 (3H, s), 3.59 (2H, t), 3.46 (2H, t), 2.37 (2H, t), 2.03 (2H, dt). [M+H]⁺ = 242.1

### Example 2. Controlled release compositions of 2-(2,5-dioxopyrrolidin-1-yl)ethyl methyl fumarate (Compound 1).

The source of various materials and equipment is indicated throughout the Example. Where a source is not indicated the material or equipment would be readily available to the skilled person. In the Example that follows: "EP" means European Pharmacopeia; "NF" means National Formulary; and "USP" means US Pharmacopeia.

### Example 2.1.

### 2.1.1 Compound 1 mini-tablet cores (uncoated)

Mini-tablet cores for use in compositions according to the invention were prepared using the materials set out in Table 2.1.1 below.

**Table 2.1.1. Mini-tablet cores of Compound 1.**

| Material | Amount (mg/mini-tab) | Amount (% (w/w)) |
|---|---|---|
| | | |
| Compound 1 | 7.00 | 87.50 |
| Microcrystalline cellulose (Avicel® PH102) | 0.36 | 4.50 |
| Crospovidone | 0.40 | 5.00 |
| Colloidal silicon dioxide | 0.16 | 2.00 |
| Magnesium stearate (non-bovine) | 0.08 | 1.00 |
| Total | 8.00 | 100.00 |

Mini-tablet cores were manufactured on a 4.5 kg scale as follows:
1. Blending: The prodrug (Compound 1), colloidal silicon dioxide and crospovidone were passed through a 500 micron screen and charge to a 25 L v-shell blender. The mixture was blended for 15 minutes at 18 rpm. The magnesium stearate was then added followed by further blending for an additional 5 minutes at 18 rpm.
2. Compression: The blend from the previous step was compressed into mini-tablets using a Riva PICCOLA, 8 station, tablet press (Riva Europe - Aldershot, UK) setup with single tip punches, each punch having a 2 mm plain normal concave tip. The resultant mini-tablets had a weight of approximately 8 mg (range 7.2 to 8.8 mg), thickness of approximately 2.00 mm (+ / - 5%) and hardness of approximately 9 N.

### 2.1.2 Compound 1 delayed release mini-tablets

A coating dispersion was prepared by dissolving Eudragit L100-55 (0.210 kg) in a 60:40 mixture of isopropyl alcohol (IPA) and water (2.644 kg) and mixing in triethyl citrate (0.042 kg), colloidal silicon dioxide (0.042 g) and talc (0.042 kg). The coating solution was applied to mini-tablet cores from Section 2.1.1 using a Vector LDCS-3 coater (Freund-Vector Corp, Iowa, USA). The coating was applied to achieve a 15 % weight gain, corresponding to a coating thickness of approximately 40-60 µm.

The final composition of the DR mini-tablets is shown in Table 2.1.2. The mini-tabs were then filled in size OO HPMC capsules to give the desired strength of Compound 1. For example, appropriate numbers of mini-tablets were filled into 1, 2 or 3 OO capsules to achieve dosage strengths of 49 mg, 70 mg, 105 mg, 210 mg, 420 mg, 455 mg, 630 mg, 840 mg, 980 mg 1120 mg and 1470 mg (all weights being mg of prodrug).

**Table 2.1.2. DR minitab composition. ^{[1]} Prepared as per Example 1; *Purified water & isopropyl alcohol are used as solvents for the DR polymer during application of coating, but if present are only in trace amounts in final composition; **USP/NF Eudragit L100-55, Evonik Industries AG, Essen, German**

| **Material** | **Source** | **Function** | **Amount** (mg/minitab) | **Amount** (% (w/w)) |
|---|---|---|---|---|
| | | | | |

| *Minitablet Core* | | | | |
|---|---|---|---|---|
| Compound 1 | ^{[1]} | MMF prodrug | 7.00 | 76.09 |
| Microcrystalline cellulose | FMC | Diluent / binder | 0.36 | 3.91 |
| Crospovidone | BASF | Disintegrant | 0.40 | 4.35 |
| Colloidal silicon dioxide | Evonik | Flow aid | 0.16 | 1.74 |
| Magnesium stearate (non-bovine) | Coviden-Mallinkrodt | Lubricant | 0.08 | 0.87 |
| | | | | |

| *Delayed Release Coating* * | | | | |
|---|---|---|---|---|
| Methacrylic acid copolymer, Type C ** | Evonik Rohm GmbH | Delayed release polymer | 0.75 | 8.15 |
| Triethyl citrate | Jungbunzlauer | Plasticizer | 0.15 | 1.63 |
| Colloidal silicon dioxide | Evonik | Anti-tack agent | 0.15 | 1.63 |
| Talc (sterilised) | Custom Powders Ltd | Anti-tack agent | 0.15 | 1.63 |
| Total | | | 9.20 | 100.00 |

### 2.1.3 In vitro release profiles

Table 2.1.3 sets out the *in vitro* dissolution profile of encapsulated delayed release mini-tablets (455 mg Compound 1) prepared above. A validated HPLC method determined total release (i.e., combined concentrations of MMF, Compound 1 and hydrolysis products of Compound 1 other than MMF) using the following apparatus and conditions - USP Apparatus I (40-mesh basket); rotation: 150 rpm; medium: 900 mL 1.0 N HCl for the first two hours, thereafter switching to pH 6.8 phosphate buffer; temperature: 37.0 +/- 0.5 °C.

**Table 2.1.3. Dissolution data for Compound 1 delayed release mini-tablets in OO HPMC capsules. * Switched from 1.0 N hydrochloric acid to phosphate buffer (pH 6.8) after two hours.**

| Time point (hours) | % Released (Mean n=6) |
|---|---|
| 0 | 0 |
| 2* | 0 |
| 2.25 | 26 |
| 2.5 | 75 |
| 2.75 | 96 |
| 3 | 99 |
| 3.5 | 100 |
| 4 | 100 |
| 4.5 | 100 |
| 5 | 100 |

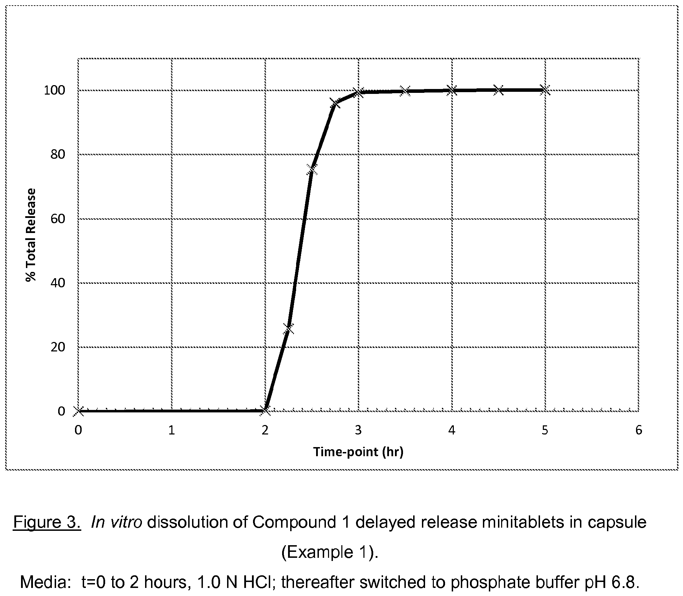

The data from Table 2.1.3 is depicted in Figure 3 from which it can be seen that following the switch from a low pH environment to phosphate buffer of pH 6.8 the prodrug is released rapidly. This indicates that the enteric coating applied to the mini-tabs protects the prodrug in the acidic medium, but readily facilitates release once the mini-tabs are switched to the phosphate buffer.

### 2.1.4 Stability

Capsules containing mini-tablets (455 mg Compound 1) prepared above were placed on stability in HDPE induction sealed bottles, without desiccant, under temperature / relative humidity conditions of 25°C / 60 %RH and 40°C / 75 %RH. Good stability was observed upon storage for three months (t=3mths), with total impurities of less than 0.2% under each set of conditions. In both cases the *in vitro* release profile of the capsules at t=3mths, tested as per the method set out in section 1.3 above, was substantially the same as that at t=0 (as detailed in Table 2.1.3 and Figure 3).

### Example 2.2.

### 2.2.1 Mini-tablet cores

Further mini-tablet cores comprising Compound 1 and having the compositions 2(a) to 2(f) set out in Table 2.2.1 were prepared according to the process set out in Section 2.1.1. There cores may be coated with a delayed release coating such as that set out in Section 2.1.2.

**Table 2.2.1. Compound 1 DR minitab core compositions 2(a) - 2(f). *Sources of materials as per Table 2.1.2. 2.2.2 DR coating levels**

| **Material** *** | **2(a)** (% (w/w)) | **2(b)** (% (w/w)) | **2(c)** (% (w/w)) | **2(d)** (% (w/w)) | **2(e)** (% (w/w)) | **2(f)** (% (w/w)) |
|---|---|---|---|---|---|---|
| | | | | | | |

| *Minitablet Core* | | | | | | |
|---|---|---|---|---|---|---|
| Compound 1 | 87.0 | 92.5 | 92.5 | 87.0 | 81.0 | 88.0 |
| Microcrystalline cellulose | 8.0 | 2.5 | 2.0 | 2.0 | 8.0 | 4.5 |
| Crospovidone | 2.0 | 2.0 | 2.5 | 8.0 | 8.0 | 4.5 |
| Colloidal silicon dioxide | 2.0 | 2.0 | 2.0 | 2.0 | 2.0 | 2.0 |
| Magnesium stearate (non-bovine) | 1.0 | 1.0 | 1.0 | 1.0 | 1.0 | 1.0 |
| | | | | | | |
| Total | 100.0 | 100.0 | 100.0 | 100.0 | 100.0 | 100.0 |

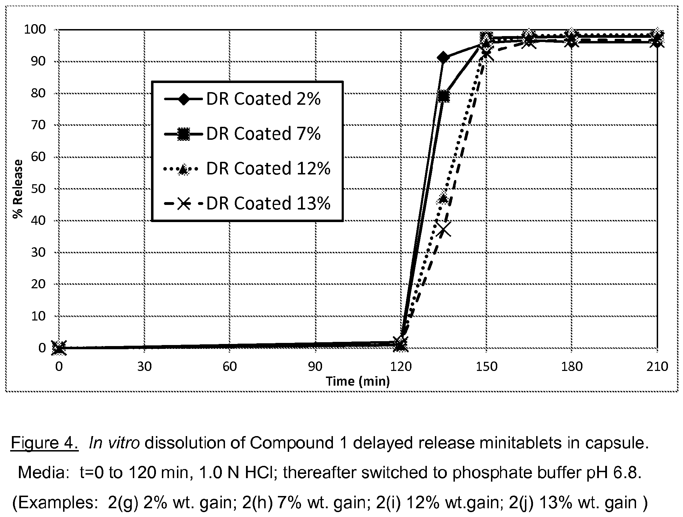

Delayed release mini-tablets comprising Compound 1 and having different levels of delayed release polymer coating were prepared using mini-tablet cores having the composition set out in Table 2.1.1. A coating dispersion was prepared as per Section 2.1.2 with the exceptions that the colloidal silicon dioxide was omitted and the solvent system employed was a 90:10 IPA / water mix. Mini-tablet cores were coated to levels of 2, 7, 12 and 13% weight gain (compositions 2(g) to 2(j) respectively). Dissolution plots (carried out according to the methodology set out in Section 2.1.3) are shown in Figure 4. From the figure it can be seen that each composition provided good enteric protection with essentially zero release occurring until the switch to pH 6.8 phosphate buffer.

### Example 2.3.

Mixtures of Compound 1 and polyethylene oxide (PEO) were extruded using a Three-Tec "Mini-Extruder" (fitted with a 12 mm twin screw and a 2 mm round die; Three-Tec GmbH, Seon, Switzerland) as per the details set out in Table 2.3.1. The extrudate strands were chopped and spheronized (Three-Tec chopper and spheronizer; Three-Tec GmbH, Seon, Switzerland) yielding spheronized extruded drug loaded cores. Extruded spheres thus produced were coated with an enteric coating (comprising Eudragit® L100-55 as per Table 2.1.2 above) to a 15 % weight gain. Extruded spheres were filled into size OO HPMC capsules to a prodrug strength of 455 mg.

**Table 2.3.1. Extrusion summary. (* Prodrug = Compound 1; Polyox™ 303 = polyethylene oxide 7,000,000 cP, Polyox™ N10 = polyethylene oxide 100,000; The Dow Chemical Company, Midland, Michigan, USA)**

| Example No. | Prodrug * / PEO ratio | PEO polymer details | Temperature (°C) Zone 1 : Zone 2 : Zone 3 | Screw speed (rpm) | Torque (Nm) |
|---|---|---|---|---|---|
| 3(a) | 90 : 10 | Polyox™ 303 | 75 : 75 : 75 | 25 to 10 | >25 |
| 3(b) | 85 : 15 | Polyox™ 303 | 75 : 75 : 75 | 25 to 10 | >25 |
| 3(c) | 85 : 15 | Polyox™ N10 | 75 : 75 : 75 | 50 | 3-4 |
| 3(d) | 90 : 10 | Polyox™ N10 | 75 : 75 : 75 | 50 | 3-4 |
| 3(e) | 85 : 15 | Polyox™ N10 | 75 : 75 : 75 | 25 | 6-9 |
| 3(f) | 90 : 10 | Polyox™ N10 | 78 : 78 : 78 | 10 | 6-9 |
| 3(g) | 90 : 10 | Polyox™ 303 | 85 : 85 : 85 | 15 | >25 |
| 3(h) | 90 : 10 | Polyox™ 303 | 96 : 96 : 96 | 10 | 12-15 |

*In vitro* dissolution of capsules containing uncoated and coated extruded spheres (455 mg prodrug) was carried out using the apparatus, methodology and conditions described in Section 2.1.3 above, with the exception that a rotation speed of 100 rpm was employed (rather than 150 rpm in the case of Example 2.1).

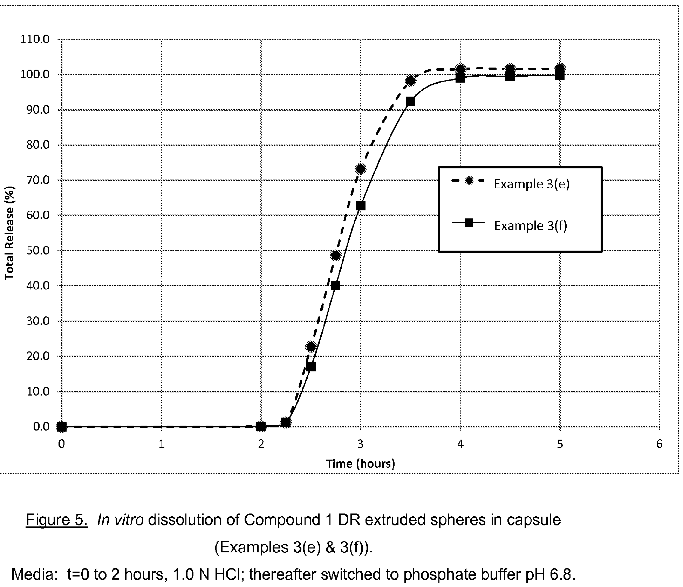

The uncoated extruded spheres were found to release substantially all of Compound 1 (and associated compounds, namely MMF and hydrolysis products of Compound 1 other than MMF) within 2 hours in phosphate buffer, pH 6.8. Dissolution profiles for enteric coated extruded spheres 3(e) and 3(f) are shown in Figure 5. As can be seen from the figure no release is observed in the acid medium, indicating the protection afforded by the enteric coating. Upon transfer to phosphate buffer there is rapid and complete release within about 2 hours. This immediate, burst release once the enteric coat has been removed is consistent with the rapid release observed for the uncoated extruded spheres.

Raw extrudate, spheronized cores and coated spheres based on composition 3(e) of Table 2.3.1 were found to have good stability upon storage at 25°C / 60 %RH and 40°C / 75 %RH for three months. In each case assay values of greater than 99.95 % and total impurities of less than 0.05 % were observed at the three month time point.

### Example 3 - Safety, Tolerability and Pharmacokinetics of Delayed Release (DR) Dosage Form Containing Compound 1 (Part 1)

A randomized, double-blind, placebo-controlled, sequential, single ascending dose study was conducted in healthy subjects to investigate the safety, tolerability and pharmacokinetics of capsules containing delayed release (DR) mini-tablets of Compound 1 from Example 2.1 ("Compound 1 DR capsules"). Up to 7 cohorts were included with 8 subjects in each cohort. In each cohort, 6 subjects received Compound 1 DR capsules and 2 subjects were treated with placebo in fasted condition. Compound 1 doses investigated were 49 mg (Cohort 1), 105 mg (Cohort 2), 210 mg (Cohort 3), 420 mg (Cohort 4), 630 mg (Cohort 5), 840 mg (Cohort 6), and 980 mg (Cohort 7) (all weights being mg of prodrug).

Blood samples were collected from all subjects prior to dosing and at 0.25, 0.50, 1, 1.50, 2, 2.50, 3, 4, 6, 8, 10, 12, and 24 hours after dosing. Plasma obtained from blood samples were analyzed for Compound 1, MMF and another metabolite using validated LC-MS/MS method. The plasma concentration-time profiles were analyzed via Non-Compartmental analysis (NCA) using Phoenix WinNonLin, version 6.3.

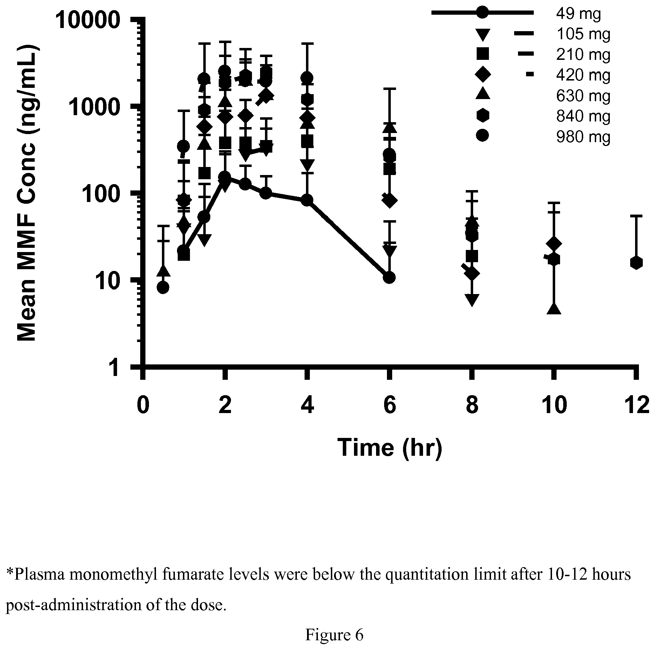

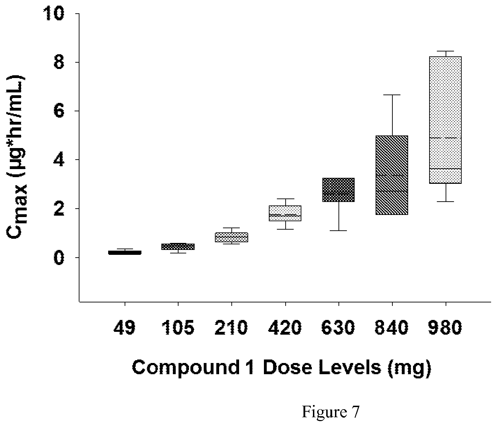

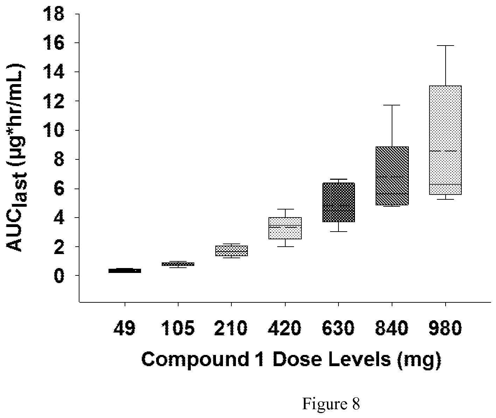

The mean plasma concentrations of MMF following oral dosing of Compound 1 are shown in Figure 6. The pharmacokinetic parameters Cₘₐₓ and AUCₗₐₛₜ among subjects administered Compound 1 are shown in Figure 7 and Figure 8. Table 3.1 summarizes the pharmacokinetic parameters for each Compound 1 dose level investigated. The drug was well tolerated during the trial and all 56 subjects completed the study. Compound 1 was rapidly converted to its metabolite MMF in plasma. MMF exposure increased with increasing Compound 1 dose levels.

There were no deaths, no serious adverse events (AEs), no severe AEs and no AEs leading to discontinuation. All AEs were mild except for two moderate events (one event of flushing at 840 mg and one event of presyncope associated with an event of mild orthostatic hypotension at 980 mg). The most common AEs were flushing and gastrointestinal (GI)-related AEs. Flushing occurred in >50% of subjects at dose levels ≥420 mg and GI-related AEs occurred most notably at the highest dose level of 980 mg. See Table 3.2. There were no clinically significant findings for labs, ECGs or vital signs (excluding the event of presyncope/orthostatic hypotension).

**Table 3.1**

| **PK Parameter (Unit)** | **Dose Levels (mg)** | | | | | | |
|---|---|---|---|---|---|---|---|
| | **49** | **105** | **210** | **420** | **630** | **840** | **980** |
| **Cmax (µg/mL)** | 0.21 (0.09) | 0.45 (0.16) | 0.84 (0.24) | 1.78 (0.42) | 2.61 (0.79) | 3.35 (1.92) | 4.90 (2.68) |
| **AUCₗₐₛₜ (µg· hr/mL)** | 0.34 (0.11) | 0.79 (0.15) | 1.70 (0.38) | 3.34 (0.90) | 4.80 (1.39) | 6.78 (2.68) | 8.57 (4.33) |
| **^{a}AUC_{0-inf} (µg· hr/mL)** | 0.53 | -- | 1.75 (0.29) | 3.00 (0.90) | 4.84 (1.02) | 6.61 (2.91) | 7.53 (3.11) |
| **^{b}Tₘₐₓ (hr)** | 2.25 (2-4) | 3 (2.5 - 4) | 3.5 (1.5 - 6) | 2.5 (1.5 - 3) | 2.5 (2 - 6) | 2.5 (2 - 3) | 2.5 (1.5 - 4) |
| **^{b}t_{1/2}** (hr) | 0.63 | -- | 0.70 (0.56 - 0.84) | 0.83 (0.53 - 1.43) | 0.76 (0.53 - 1.17) | 0.79 (0.76-1.03) | 0.68 (0.59 - 0.92) |
| **^{b}T_{lag} (hr**) | 1.25 (0.5 - 1.5) | 1.5 (0.5 - 2) | 1.75 (0.5-3) | 1 (0.5 - 1.5) | 0.5 (0.25 - 3) | 0.75 (0.5 - 2) | 0.5 (0.25 - 1) |
| **^{b}Tₗₐₛₜ (hr)** | 4 (3 - 6) | 5 (4 - 8) | 7 (4 - 8) | 6 (4 - 10) | 8 (6 - 10) | 8 (6 - 12) | 7 (6 - 8) |

| | | | | | | | |
|---|---|---|---|---|---|---|---|
| Mean (SD) of N = 6 subjects ^{a}Mean of N = 1 to 5 subjects ^{b}Median (Range) | | | | | | | |

**Table 3.2. Subjects with GI-related and Flushing AEs by Dose Level (Part 1)**

| | | **Compound 1** | | | | | | |
|---|---|---|---|---|---|---|---|---|
| **Adverse Event System Organ Class and Related Preferred Term(s)** | **Placebo (N=14) n(%)** | **49 mg (N=6) n(%)** | **105 mg (N=6) n(%)** | **210 mg (N=6) n(%)** | **420 mg (N=6) n(%)** | **630 mg (N=6) n(%)** | **840 mg (N=6) n(%)** | **980 mg (N=6) n(%)** |
| **Gastrointestina 1 disorders (SOC)** | 0 | 1 (16.7) | 0 | 1 (16.7) | 0 | 1 (16.7) | 2 (33.3) | 4(66.7) |
| Diarrhoea | 0 | 0 | 0 | 0 | 0 | 1 (16.7) | 1 (16.7) | 2 (33.3) |
| Flatulence | 0 | 0 | 0 | 0 | 0 | 0 | 2 (33.3) | 1 (16.7) |
| Abdominal discomfort | 0 | 0 | 0 | 1 (16.7) | 0 | 0 | 0 | 1 ( 16.7) |
| Nausea | 0 | 0 | 0 | 0 | 0 | 0 | 0 | 2 (33.3) |
| Abdominal pain | 0 | 0 | 0 | 0 | 0 | 0 | 0 | 1 (16.7) |
| Frequent bowel movements | 0 | 1 (16.7) | 0 | 0 | 0 | 0 | 0 | 0 |
| **Vascular disorders (SOC)** | 0 | 1 (16.7) | 0 | 2 (33.3) | 4 (66.7) | 6 (100.0) | 5 (83.3) | 6 (100.0) |
| Flushing | 0 | 1 (16.7) | 0 | 2 (33.3) | 4 (66.7) | 6 (100.0) | 5 (83.3) | 6 (100.0) |

| | | | | | | | | |
|---|---|---|---|---|---|---|---|---|
| Note: SOC = System Organ Class | | | | | | | | |

### Example 4- A Phase I study to compare the safety, tolerability, and pharmacokinetics of a 420 mg Compound 1 Delayed Release (DR) dosage form versus 240 mg dimethyl fumarate (DMF) in healthy subjects (Part 2)

A randomized, double-blind, placebo-controlled, 2-treatment, 2-period cross-over study was conducted to investigate the safety, tolerability, and pharmacokinetics of Compound 1 DR and DMF oral dosage forms. (The DMF oral dosage form used in this study was TECFIDERA®, (Biogen Idec, Inc Cambridge, MA, USA)). A total of 16 subjects were enrolled and randomized to a treatment sequence.

In sequence 1, six subjects were given orally 420 mg Compound 1 DR (Dosing Period 1), followed by 240 mg DMF (Dosing Period 2) under fasting condition. Each dosing period was separated by a wash-out period of 7 days.

In sequence 2, six subjects were given orally 240 mg DMF (Dosing Period 1), followed by 420 mg Compound 1 DR (Dosing Period 2) under fasting condition. Each dosing period was separated by a wash-out period of 7 days.

In sequence 3, four subjects received placebo treatment during both dosing periods (Period 1 and Period 2) under fasting condition. Each dosing period was separated by a wash-out period of 7 days.

Blood samples were collected from all subjects prior to dosing and at 0.25, 0.50, 1, 1.50, 2, 2.50, 3, 4, 6, 8, 10, 12, and 24 hours after dosing. Plasma obtained from blood samples were analyzed for Compound 1, MMF and another metabolite using validated LC-MS/MS method. The plasma concentration-time profiles were analyzed via Non-Compartmental analysis (NCA) using Phoenix WinNonLin, version 6.3.

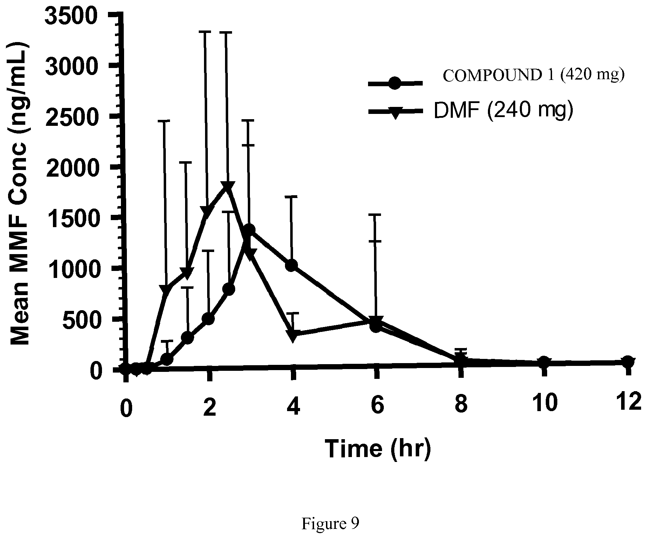

All subjects in both treatment sequences completed the study. The mean plasma concentrations of MMF following both treatments are shown in Figure 9 Table 4.1 summarizes the pharmacokinetic parameters for both the treatment groups. The maximum plasma concentration (Cₘₐₓ) of MMF was decreased by approximately 34% in the Compound 1 treatment group in comparison to DMF. The median lag time for absorption (T_{lag}) was longer for Compound 1 DR dosage form (1.5 hr) when compared to DMF (0.5 hr). However, the MMF exposure (as measured by AUCₗₐₛₜ) was comparable between both the dosage forms. Further, treatment with Compound 1 dosage form resulted in less PK variability when compared to DMF treatment as evident from the relatively lower %CV values (Table 4.1).

There were no deaths, no serious AEs, no severe AEs and no AEs leading to discontinuation. All AEs were mild except for two moderate events (one event of flushing and one event of retching in two subjects treated with DMF). The most common AEs were flushing and GI-related AEs. See Table 4.2. Flushing occurred in 8 (66.7%) of subjects treated with DMF and 8 (66.7%) of subjects treated with Compound 1. In contrast, GI-related AEs occurred in 5 (41.7%) of subjects treated with DMF compared to 1 (8.3%) treated with Compound 1. There were no clinically significant findings for labs, ECGs or vital signs.

The single GI-related AE in the subject treated with Compound 1 was an event of constipation that occurred approximately 40 hours after dosing and was regarded by the investigator to be unrelated to study drug. Subjects experiencing notable GI-related AEs only occurred after treatment with DMF and included 3 (25%) subjects with nausea, 1 (8.3%) subject with diarrhea, and 1 (8.3%) subject with retching (1 subject experienced all three events).

Consistent with the finding of a longer T_{lag} with Compound 1 DR compared to DMF, there was a delay in the event of flushing with Compound 1 DR (mean onset 2.6 hours) compared to DMF (mean onset 1.2 hours).

**Table 4.1**

| **PK Parameter (Unit)** | **Treatment Group (N=12)** | | | |
|---|---|---|---|---|
| | **Compound 1 (420 mg)** | | **DMF (240 mg)** | |
| | **Mean (SD)** | **%CV** | **Mean (SD)** | **%CV** |
| **Cₘₐₓ (µg/mL)** | 2.04 (0.74) | 36.5 | 3.11 (1.52) | 48.9 |
| **AUCₗₐₛₜ (µg· hr/mL)** | 4.15 (1.28) Median: 4.18 | 30.9 | 4.78 (2.06) Median: 4.20 | 43.1 |
| **^{a}AUC_{0-inf} (µg· hr/mL)** | 3.73 (0.92) Median: 3:82 | 24.7 | 4.90 (2.14) Median: 4.41 | 43.7 |
| **^{b}Tₘₐₓ (hr)** | 3 (2 - 6) | 34.0 | 2.5 (1 - 6) | 60.7 |
| **^{b}t_{1/2} (hr)** | 0.73 (0.51 - 1.18) | 31.4 | 0.63 (0.48 - 0.87) | 23.9 |
| **^{b}T_{lag} (hr)** | 1.5 (0.25 - 4) | 67.8 | 0.5 (0 - 3) | 114.7 |
| **^{b}Tₗₐₛₜ (hr)** | 7 (6 - 10) | 18.7 | 6(4 - 10) | 34.8 |

| | | | | |
|---|---|---|---|---|
| ^{a}Mean of N = 5 to 8 subjects ^{b}Median (Range) | | | | |

**Table 4.2. Subjects with GI-related and Flushing AEs by Treatment Group (Part 2)**

| **Adverse Event System Organ Class and Related Preferred Term(s)** | Placebo (N=4) n (%) | DMF 240 mg (N=12) n (%) | Compound 1 DR 420 mg (N=12) n (%) |
|---|---|---|---|
| **Gastrointestinal disorders (SOC)** | 0 | 5 (41.7) | 1 (8.3) |
| Constipation | 0 | 0 | 1 (8.3) |
| Diarrhoea | 0 | 1 (8.3) | 0 |
| Eructation | 0 | 1 (8.3) | 0 |
| Flatulence | 0 | 1 (8.3) | 0 |
| Nausea | 0 | 3 (25.0) | 0 |
| Retching | 0 | 1 (8.3) | 0 |
| **Vascular disorders (SOC)** | 0 | 8 (66.7) | 8 (66.7) |
| Flushing | 0 | 8 (66.7) | 8 (66.7) |

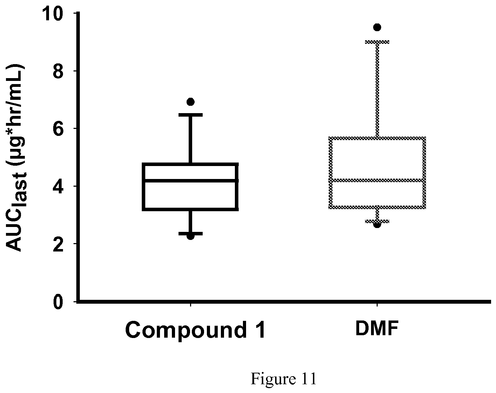

Plasma monomethyl fumarate exposure (AUCₗₐₛₜ) was comparable between cohort 1 and cohort 2. Surprisingly, although the dose equivalent of monomethyl fumarate was essentially identical, the Cₘₐₓ of monomethyl fumarate was decreased by approximately 34% following administration of 2-(2,5-dioxopyrrolidin-1-yl)ethyl methyl fumarate in comparison to dimethyl fumarate. Furthermore, less variability in the pharmacokinetic parameters Cₘₐₓ and AUCₗₐₛₜ among subjects administered 2-(2,5-dioxopyrrolidin-1-yl)ethyl methyl fumarate, as compared to subjects administered dimethyl fumarate, was observed (Figure 10 and Figure 11). In addition, the median lag time for absorption was 1.5 hours for subjects administered 2-(2,5-dioxopyrrolidin-1-yl)ethyl methyl fumarate compared to 0.5 hours for those administered dimethyl fumarate (Figure 9).

## Claims

1. A pharmaceutical composition for once or twice daily administration of a monomethyl fumarate (MMF) prodrug, wherein said MMF prodrug is 2-(2,5-dioxopyrrolidin-1-yl)ethyl methyl fumarate or a pharmaceutically acceptable salt thereof, said composition comprising 2-(2,5-dioxopyrrolidin-1-yl)ethyl methyl fumarate or a pharmaceutically acceptable salt thereof and a controlled release polymer, wherein the controlled release polymer is in the form of a coating applied to a core containing the 2-(2,5-dioxopyrrolidin-1-yl)ethyl methyl fumarate or a pharmaceutically acceptable salt thereof.

2. A pharmaceutical composition according to claim 1 wherein the core is a tablet or pellet comprising 2-(2,5-dioxopyrrolidin-1-yl)ethyl methyl fumarate or a pharmaceutically acceptable salt thereof, preferably, wherein the pharmaceutical composition comprises a plurality of tablets or pellets.

3. A pharmaceutical composition according to claim 1 or 2, wherein the coating is an enteric coating.

4. A pharmaceutical composition according to claim 3 wherein
a) the 2-(2,5-dioxopyrrolidin-1-yl)ethyl methyl fumarate or a pharmaceutically acceptable salt thereof is released substantially immediately following removal of the enteric coating; or
b) substantially all of the 2-(2,5-dioxopyrrolidin-1-yl)ethyl methyl fumarate or a pharmaceutically acceptable salt thereof is released from the composition within about 2 hours in pH 6.8 as measured using USP apparatus I 40-mesh basket at a rotation speed of from 100 to 150 rpm; preferably 100 % of the 2-(2,5-dioxopyrrolidin-1-yl)ethyl methyl fumarate or a pharmaceutically acceptable salt thereof is released from the composition within about 2 hours in pH 6.8 as measured using USP apparatus I 40-mesh basket at a rotation speed of from 100 to 150 rpm.

5. A pharmaceutical composition according to any preceding claim wherein the 2-(2,5-dioxopyrrolidin-1-yl)ethyl methyl fumarate or a pharmaceutically acceptable salt thereof is dispersed throughout a carrier matrix to form a plurality of pellets, preferably wherein the pellets are produced by melt extrusion, and subsequently coated with the controlled release polymer.

6. A pharmaceutical composition according to any preceding claim wherein the core further comprises a diluent and a disintegrant.

7. A pharmaceutical composition consisting essentially of a core comprising 2-(2,5-dioxopyrrolidin-1-yl)ethyl methyl fumarate or a pharmaceutically acceptable salt thereof, a diluent and a disintegrant; and a coating comprising a controlled release polymer.

8. A pharmaceutical composition according to any preceding claim wherein the coating further comprises a plasticizer and one or more anti-tack agents.

9. A pharmaceutical composition according to claim 6 or claim 7 wherein the diluent is selected from the group consisting of microcrystalline cellulose, dextrose, lactose, sucrose, mannitol, dicalcium phosphate and combinations thereof; wherein the disintegrant is selected from the group consisting of sodium carboxymethylcellulose, starch, crosslinked polyvinylpyrrolidone (crospovidone) and combinations thereof; wherein the controlled release polymer is an enteric polymer; and wherein the coating further comprises (a) plasticizer selected from the group consisting of triacetin, tributyl citrate, triethyl citrate, dibutyl sebacate, diethyl phthalate and combinations thereof, and (b) one or more anti-tack agents selected from the group consisting of colloidal silicon dioxide, talc and combinations thereof.

10. A pharmaceutical composition according to claim6 or claim 7, wherein the diluent is microcrystalline cellulose and the coating comprises methacrylic acid copolymer - Type C as the controlled release polymer; preferably wherein the core further comprises crospovidone or magnesium stearate.

11. A pharmaceutical composition according to any preceding claim wherein the pharmaceutical composition comprises a plurality of tablets and the controlled release polymer is applied to the tablets at a level of from about 2 to about 30 % weight gain or from about 0.95 to about 14.75 mg/cm²; or comprises a plurality of tablets wherein the coating has a thickness of from about 40 to about 60 microns.

12. A pharmaceutical composition according to any preceding claim wherein the composition consists of 70% to 80% 2-(2,5-dioxopyrrolidin-1-yl)ethyl methyl fumarate, by weight.

13. A pharmaceutical composition according to any preceding claim wherein the core consists of:
| Material | Amount (% (w/w)) |
|---|---|
| 2-(2,5-dioxopyrrolidin-1-yl)ethyl methyl fumarate | 76.09 |
| microcrystalline cellulose | 3.91 |
| crospovidone | 4.35 |
| colloidal silicon dioxide | 1.74 |
| magnesium stearate (non-bovine) | 0.87 |
further wherein the coating consists of:
| Material | Amount (% (w/w)) |
|---|---|
| methacrylic acid copolymer - Type C | 8.15 |
| triethyl citrate | 1.63 |
| colloidal silicon dioxide | 1.63 |
| Talc (sterilised) | 1.63 |
; and further wherein the amount (w/w) represents the percentage weight of the pharmaceutical composition.

14. A solid oral dosage form, wherein the solid oral dosage form is a capsule containing the pharmaceutical composition of claim 2, 5 or 11, said pharmaceutical composition comprising a plurality of said tablets or pellets.

15. A pharmaceutical composition or solid oral dosage form of any preceding claim for use in a method of treating:
a) multiple sclerosis, comprising administering to a subject in need thereof a therapeutically effective amount of said pharmaceutical composition or solid oral dosage form, optionally wherein the method reduces the probability of an occurrence of a drug-induced gastrointestinal disorder in a subject who is currently being treated with dimethyl fumarate or who is contemplating treatment with dimethyl fumarate; or
b) a neurological disorder in a subject, comprising administering to the subject said pharmaceutical composition or solid oral dosage form.

## Patentansprüche

1. Pharmazeutische Zusammensetzung zur einmaligen oder zweimaligen täglichen Verabreichung eines Monomethylfumarat(MMF)-Prodrugs, wobei es sich bei dem MMF-Prodrug um 2-(2,5-Dioxopyrrolidin-1-yl)ethylmethylfumarat oder ein pharmazeutisch unbedenkliches Salz davon handelt, wobei die Zusammensetzung 2-(2,5-Dioxopyrrolidin-1-yl)ethylmethylfumarat oder ein pharmazeutisch unbedenkliches Salz davon und ein Retardpolymer umfasst, wobei das Retardpolymer in Form eines Überzugs, der auf einen das 2-(2,5-Dioxopyrrolidin-1-yl)ethylmethylfumarat oder ein pharmazeutisch unbedenkliches Salz davon enthaltenden Kern aufgebracht ist, vorliegt.

2. Pharmazeutische Zusammensetzung nach Anspruch 1, wobei es sich bei dem Kern um eine Tablette oder ein Pellet, das 2-(2,5-Dioxopyrrolidin-1-yl)ethylmethylfumarat oder ein pharmazeutisch unbedenkliches Salz davon umfasst, handelt, wobei die pharmazeutische Zusammensetzung vorzugsweise mehrere Tabletten oder Pellets umfasst.

3. Pharmazeutische Zusammensetzung nach Anspruch 1 oder 2, wobei es sich bei dem Überzug um einen magensaftresistenten Überzug handelt.

4. Pharmazeutische Zusammensetzung nach Anspruch 3, wobei
a) das 2-(2,5-Dioxopyrrolidin-1-yl)ethylmethylfumarat oder ein pharmazeutisch unbedenkliches Salz davon im Wesentlichen unmittelbar nach dem Entfernen des magensaftresistenten Überzugs freigesetzt wird; oder
b) im Wesentlichen das gesamte 2-(2,5-Dioxopyrrolidin-1-yl)ethylmethylfumarat oder ein pharmazeutisch unbedenkliches Salz davon bei einem pH-Wert von 6,8 gemäß Messung unter Verwendung einer USP-Vorrichtung mit I-40-Gitterkorb bei einer Drehgeschwindigkeit von 100 bis 150 U/min innerhalb von etwa 2 Stunden aus der Zusammensetzung freigesetzt wird; vorzugsweise 100 % des 2-(2,5-Dioxopyrrolidin-1-yl)ethylmethylfumarats oder eines pharmazeutisch unbedenklichen Salzes davon bei einem pH-Wert von 6, 8 gemäß Messung unter Verwendung einer USP-Vorrichtung mit I-40-Gitterkorb bei einer Drehgeschwindigkeit von 100 bis 150 U/min innerhalb von etwa 2 Stunden aus der Zusammensetzung freigesetzt werden.

5. Pharmazeutische Zusammensetzung nach einem der vorhergehenden Ansprüche, wobei das 2-(2,5-Dioxopyrrolidin-1-yl)ethylmethylfumarat oder ein pharmazeutisch unbedenkliches Salz davon in der gesamten Trägermatrix zur Bildung mehrerer Pellets dispergiert ist, wobei die Pellets vorzugsweise durch Schmelzeextrusion hergestellt werden und anschließend mit dem Retardpolymer überzogen werden.

6. Pharmazeutische Zusammensetzung nach einem der vorhergehenden Ansprüche, wobei der Kern ferner ein Verdünnungsmittel und ein Sprengmittel umfasst.

7. Pharmazeutische Zusammensetzung, die im Wesentlichen aus einem Kern, der 2-(2,5-Dioxopyrrolidin-1-yl)ethylmethylfumarat oder ein pharmazeutisch unbedenkliches Salz davon, ein Verdünnungsmittel und ein Sprengmittel enthält; und einem ein Retardpolymer umfassenden Überzug besteht.

8. Pharmazeutische Zusammensetzung nach einem der vorhergehenden Ansprüche, wobei der Überzug ferner einen Weichmacher und ein oder mehrere Trennmittel umfasst.

9. Pharmazeutische Zusammensetzung nach Anspruch 6 oder Anspruch 7, wobei das Verdünnungsmittel aus der Gruppe bestehend aus mikrokristalliner Cellulose, Dextrose, Lactose, Saccharose, Mannit, Dicalciumphosphat und Kombinationen davon ausgewählt ist; wobei das Sprengmittel aus der Gruppe bestehend aus Natriumcarboxymethylcellulose, Stärke, vernetztem Polyvinylpyrrolidon (Crospovidon) und Kombinationen davon ausgewählt ist; wobei es sich bei dem Retardpolymer um ein magensaftresistentes Polymer handelt; und wobei der Überzug ferner (a) einen aus der Gruppe bestehend aus Triacetin, Citronensäuretributylester, Citronensäuretriethylester, Sebacinsäuredibutylester, Phthalsäurediethylester und Kombinationen davon ausgewählten Weichmacher und (b) ein oder mehrere aus der Gruppe bestehend aus kolloidalem Siliciumdioxid, Talk und Kombinationen davon ausgewählte Trennmittel umfasst.

10. Pharmazeutische Zusammensetzung nach Anspruch 6 oder Anspruch 7, wobei es sich bei dem Verdünnungsmittel um mikrokristalline Cellulose handelt und der Überzug Methacrylsäure-Copolymer vom Typ C als das Retardpolymer umfasst; wobei der Kern ferner vorzugsweise Crospovidon oder Magnesiumstearat umfasst.

11. Pharmazeutische Zusammensetzung nach einem der vorhergehenden Ansprüche, wobei die pharmazeutische Zusammensetzung mehrere Tabletten umfasst und das Retardpolymer auf die Tabletten mit einem Gehalt von etwa 2 bis etwa 30 % Gewichtszunahme oder von etwa 0,95 bis etwa 14,75 mg/cm² aufgebracht ist; oder mehrere Tabletten umfasst, wobei der Überzug eine Dicke von etwa 40 bis etwa 60 Mikron aufweist.

12. Pharmazeutische Zusammensetzung nach einem der vorhergehenden Ansprüche, wobei die Zusammensetzung aus 70 Gew.-% bis 80 Gew.-% 2-(2,5-Dioxopyrrolidin-1-yl)ethylmethylfumarat besteht.

13. Pharmazeutische Zusammensetzung nach einem der vorhergehenden Ansprüche, wobei der Kern aus Folgendem besteht:
| Stoff | Menge (% (w/w)) |
|---|---|
| 2-(2,5-Dioxopyrrolidin-1-yl)ethylmethylfumarat | 76,09 |
| Mikrokristalline Cellulose | 3,91 |
| Crospovidon | 4,35 |
| Kolloidales Siliciumdioxid | 1,74 |
| Magnesiumstearat (nicht bovin) | 0,87 |
wobei der Überzug ferner aus Folgendem besteht:
| Stoff | Menge (% (w/w)) |
|---|---|
| Methacrylsäure-Copolymer vom Typ C | 8, 15 |
| Citronensäuretriethylester | 1, 63 |
| Kolloidales Siliciumdioxid | 1, 63 |
| Talk (sterilisiert) | 1, 63 |
; und wobei die Menge (w/w) ferner für den Gewichtsprozentanteil der pharmazeutischen Zusammensetzung steht.

14. Feste orale Dosierungsform, wobei es sich bei der festen oralen Dosierungsform um eine Kapsel handelt, die die pharmazeutische Zusammensetzung nach Anspruch 2, 5 oder 11 enthält, wobei die pharmazeutische Zusammensetzung mehrere der Tabletten oder der Pellets umfasst.

15. Pharmazeutische Zusammensetzung oder feste orale Dosierungsform nach einem der vorhergehenden Ansprüche zur Verwendung bei einem Verfahren zur Behandlung von:
a) multipler Sklerose, bei dem man einem dessen bedürfenden Individuum eine therapeutisch wirksame Menge der pharmazeutischen Zusammensetzung oder festen oralen Dosierungsform verabreicht, wobei durch das Verfahren gegebenenfalls die Wahrscheinlichkeit für das Auftreten einer durch Arzneistoff induzierten gastrointestinalen Störung bei einem Individuum, das gegenwärtig mit Dimethylfumarat behandelt wird oder das eine Behandlung mit Dimethylfumarat abwägt, verringert wird; oder
b) einer neurologischen Störung bei einem Individuum, bei dem man dem Individuum die pharmazeutische Zusammensetzung oder feste orale Dosierungsform verabreicht.

## Revendications

1. Composition pharmaceutique pour administration une fois ou deux fois par jour d'un promédicament de fumarate monométhylique (MMF), dans laquelle ledit promédicament de MMF est le fumarate méthylique de 2-(2,5-dioxopyrrolidin-1-yl)éthyle ou un sel pharmaceutiquement acceptable de celui-ci, ladite composition comprenant du fumarate méthylique de 2-(2,5-dioxopyrrolidin-1-yl)éthyle ou un sel pharmaceutiquement acceptable de celui-ci et un polymère à libération contrôlée, dans laquelle le polymère à libération contrôlée est sous la forme d'un enrobage appliqué sur un noyau contenant le fumarate méthylique de 2-(2,5-dioxopyrrolidin-1-yl)éthyle ou un sel pharmaceutiquement acceptable de celui-ci.

2. Composition pharmaceutique selon la revendication 1 dans laquelle le noyau est un comprimé ou un granule comprenant du fumarate méthylique de 2-(2,5-dioxopyrrolidin-1-yl)éthyle ou un sel pharmaceutiquement acceptable de celui-ci, de préférence, où la composition pharmaceutique comprend une pluralité de comprimés ou granules.

3. Composition pharmaceutique selon la revendication 1 ou 2, dans laquelle l'enrobage est un enrobage entérique.

4. Composition pharmaceutique selon la revendication 3 dans laquelle
a) le fumarate méthylique de 2-(2,5-dioxopyrrolidin-1-yl)éthyle ou un sel pharmaceutiquement acceptable de celui-ci est libéré sensiblement immédiatement après le retrait de l'enrobage entérique ; ou
b) sensiblement tout le fumarate méthylique de 2-(2,5-dioxopyrrolidin-1-yl)éthyle ou un sel pharmaceutiquement acceptable de celui-ci est libéré de la composition en environ 2 heures à pH 6,8 comme mesuré au moyen d'un appareil USP I à panier de 40 mesh à une vitesse de rotation de 100 à 150 tours/min ; de préférence 100 % du fumarate méthylique de 2-(2,5-dioxopyrrolidin-1-yl)éthyle ou un sel pharmaceutiquement acceptable de celui-ci est libéré de la composition en environ 2 heures à pH 6,8 comme mesuré au moyen d'un appareil USP I à panier de 40 mesh à une vitesse de rotation de 100 à 150 tours/min.

5. Composition pharmaceutique selon l'une quelconque des revendications précédentes dans laquelle le fumarate méthylique de 2-(2,5-dioxopyrrolidin-1-yl)éthyle ou un sel pharmaceutiquement acceptable de celui-ci est dispersé dans l'ensemble d'une matrice de support pour former une pluralité de granules, de préférence dans laquelle les granules sont produits par extrusion à l'état fondu, et ensuite enrobés avec le polymère à libération contrôlée.

6. Composition pharmaceutique selon l'une quelconque des revendications précédentes dans laquelle le noyau comprend en outre un diluant et un délitant.

7. Composition pharmaceutique essentiellement constituée d'un noyau comprenant du fumarate méthylique de 2-(2,5-dioxopyrrolidin-1-yl)éthyle ou un sel pharmaceutiquement acceptable de celui-ci, un diluant et un délitant ; et un enrobage comprenant un polymère à libération contrôlée.

8. Composition pharmaceutique selon l'une quelconque des revendications précédentes dans laquelle l'enrobage comprend en outre un plastifiant et un ou plusieurs agents antiadhésifs.

9. Composition pharmaceutique selon la revendication 6 ou la revendication 7 dans laquelle le diluant est choisi dans le groupe constitué de la cellulose microcristalline, du dextrose, du lactose, du saccharose, du mannitol, du phosphate dicalcique et des combinaisons de ceux-ci ; dans laquelle le délitant est choisi dans le groupe constitué de la carboxyméthylcellulose sodique, de l'amidon, de la polyvinylpyrrolidone réticulée (crospovidone) et des combinaisons de celles-ci ; dans laquelle le polymère à libération contrôlée est un polymère entérique ; et dans laquelle l'enrobage comprend en outre (a) un plastifiant choisi dans le groupe constitué de la triacétine, du citrate de tributyle, du citrate de triéthyle, du sébacate de dibutyle, du phtalate de diéthyle et des combinaisons de ceux-ci, et (b) un ou plusieurs agents antiadhésifs choisis dans le groupe constitué du dioxyde de silicium colloïdal, du talc et des combinaisons de ceux-ci.

10. Composition pharmaceutique selon la revendication 6 ou la revendication 7, dans laquelle le diluant est la cellulose microcristalline et l'enrobage comprend un copolymère d'acide méthacrylique de type C en tant que polymère à libération contrôlée ; de préférence dans laquelle le noyau comprend en outre de la crospovidone ou du stéarate de magnésium.

11. Composition pharmaceutique selon l'une quelconque des revendications précédentes où la composition pharmaceutique comprend une pluralité de comprimés et le polymère à libération contrôlée est appliqué sur les comprimés à un taux d'environ 2 à environ 30 % de gain de poids ou d'environ 0,95 à environ 14,75 mg/cm² ; ou comprend une pluralité de comprimés dans lesquels l'enrobage a une épaisseur d'environ 40 à environ 60 microns.

12. Composition pharmaceutique selon l'une quelconque des revendications précédentes où la composition est constituée de 70 % à 80 % de fumarate méthylique de 2-(2,5-dioxopyrrolidin-l-yl)éthyle, en poids.

13. Composition pharmaceutique selon l'une quelconque des revendications précédentes dans laquelle le noyau est constitué de :
| Matériau | Quantité (% (m/m)) |
|---|---|
| fumarate méthylique de 2-(2,5-dioxopyrrolidin-1-yl) éthyle | 76, 09 |
| cellulose microcristalline | 3, 91 |
| crospovidone | 4,35 |
| dioxyde de silicium colloïdal | 1,74 |
| stéarate de magnésium (non bovin) | 0, 87 |
en outre dans laquelle l'enrobage est constitué de :
| Matériau | Quantité (% (m/m)) |
|---|---|
| copolymère d'acide méthacrylique de type C | 8, 15 |
| citrate de triéthyle | 1, 63 |
| dioxyde de silicium colloïdal | 1, 63 |
| Talc (stérilisé) | 1, 63 |
; et en outre dans laquelle la quantité (m/m) représente le pourcentage en poids de la composition pharmaceutique.

14. Forme pharmaceutique orale solide, où la forme pharmaceutique orale solide est une capsule contenant la composition pharmaceutique selon la revendication 2, 5 ou 11, ladite composition pharmaceutique comprenant une pluralité desdits comprimés ou granules.

15. Composition pharmaceutique ou forme pharmaceutique orale solide selon l'une quelconque des revendications précédentes pour utilisation dans un procédé de traitement de :
a) la sclérose en plaques, comprenant l'administration à un sujet en ayant besoin d'une quantité thérapeutiquement efficace de ladite composition pharmaceutique ou forme pharmaceutique orale solide, facultativement où le procédé réduit la probabilité d'une occurrence d'un trouble gastro-intestinal d'origine médicamenteuse chez un sujet qui est actuellement traité avec du fumarate de diméthyle ou qui envisage un traitement avec le fumarate de diméthyle ; ou
b) un trouble neurologique chez un sujet, comprenant l'administration au sujet de ladite composition pharmaceutique ou forme pharmaceutique orale solide.
